# EUROPÄISCHE PATENTANMELDUNG

(11) **EP 2 426 101 A1**
(43) Veröffentlichungstag der Anmeldung: **07.03.2012**
(21) Anmeldenummer: 10172065.4
(22) Anmeldetag: 05.08.2010
(51) Int. Cl.: C07C 43/04, A61K 8/33, A61Q 1/00, A61Q 1/06, A61Q 5/12, A61Q 15/00, A61Q 17/04, A61Q 19/00

(54) **Kosmetische Zubereitungen**

(71) Anmelder: Cognis IP Management GmbH, 40589 Düsseldorf (DE)
(72) Erfinder: Dierker, Markus, DE-40593 Düsseldorf (DE); Beckedahl, Burkhard, DE-40589 Düsseldorf (DE); Kawa Rolf, DE-40789 Monheim (DE); Brüning Stefan, DE-40593 Düsseldorf (DE)

(57) **Zusammenfassung**

Gegenstand der Erfindung sind Verbindungen der allgemeinen Formel (I)

R₁-O-R₂,

wobei
- R₁ einen verzweigten Alkyl(en)rest mit 10 bis 22 C-Atomen darstellt, wobei mindestens eine Verzweigung sich in Position 1, 2, 3 oder 4 zum Sauerstoffatom befindet
- R₂ einen linearen oder verzweigten Alkyl(en)rest mit 1 bis 13 C Atomen darstellt
- und R₁ und R₂ so gewählt werden, dass die Gesamtzahl der C-Atome in Formel (I) 11 bis 23 beträgt.

Die erfindungsgemäßen Verbindungen eignen sich zur Herstellung von oder in kosmetischen und/oder pharmazeutischen Zubereitungen, insbesondere als Ölkörper.

## Beschreibung

### Gebiet der Erfindung

Die Erfindung betrifft spezielle Dialkyl(en)ether sowie ihre Verwendung in kosmetischen und/oder pharmazeutischen Zubereitungen sowie ein Verfahren zu deren Herstellung.

### Stand der Technik

An Zubereitungen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper und Emulgator-Gemische entwickelt und getestet. Dialkylether sind als kosmetische Ölkörper im Stand der Technik, beispielsweise in EP 0 815 837 A1 beschrieben. Ein kommerziell erhältlicher Dialkylether ist Di-n-octyl-ether, welcher unter dem Handelsnamen Cetiol® OE vertrieben wird.

Nachteilig an den Dialkyl(en)ethern des Standes der Technik ist ihre eingeschränkte Hautverträglichkeit. Weiterhin bestand eine Aufgabe der vorliegenden Erfindung darin, gegenüber den Verbindungen des Standes der Technik hinsichtlich der Sensorik verbesserte Verbindungen bereit zu stellen. Hierbei waren insbesondere die Leichtigkeit, ein "nichtfettendes" Hautgefühl, Weichheit, Spreitvermögen, Absorption, Verteilbarkeit und Öligkeit von Interesse. Weiterhin sollten die Verbindungen in eine Vielzahl kosmetischer Formulierungen einarbeitbar sind. Von besonderem Interesse war die Kompatibilität mit kristallinen UV-Filtern, Pigmenten sowie Antiperspirantien Salzen. Eine weiter Aufgabe hat darin bestanden Verbindungen bereit zu stellen, die sich als Ersatzstoffe für Silikonöle eignen.

### Beschreibung der Erfindung

Gegenstand der Erfindung sind Verbindungen der Formel (I)

R₁-O-R₂,

wobei
- R₁ einen verzweigten Alkyl(en)rest mit 10 bis 22 C-Atomen darstellt, wobei mindestens eine Verzweigung sich in Position 1, 2, 3 oder 4 zum Sauerstoffatom befindet
- R₂ einen linearen oder verzweigten Alkyl(en)rest mit 1 bis 13 C Atomen darstellt
- und R₁ und R₂ so gewählt werden, dass die Gesamtzahl der C-Atome in Formel (I) 11 bis 23 beträgt.

Sofern nicht anderes angegeben, handelt es sich bei der Angabe der Alkyl(en)reste immer um lineare Alkyl(en) reste, d.h. Octyl-Rest = n-Octyl, Tetradecyl = n-Tetradecyl.

### Rest R₁

Der Rest R₁ stellt einen verzweigten Alky(en)rest mit 10 bis 22 C-Atomen dar, wobei sich mindestens eine Verzweigung in Position 1, 2, 3 oder 4 zum Sauerstoffatom befindet.

Die Benennung der Positionen der Verzweigungen erfolgt immer ausgehend vom Sauerstoffatom des Ethers, an den der Alkyl(en)rest gebunden ist.

Der Begriff Alkyl(en)rest umfasst gesättigte (= Alkylreste) sowie einfach oder mehrfach ungesättigte Reste (beide als Alkylenreste bezeichnet). Eine bevorzugte Ausführungsform der Erfindung betrifft Verbindungen der Formel (I), in denen R₁ einen gesättigten Rest darstellt.

Der Rest R₁ ist verzweigt, d.h. mindestens ein C-Atome des Alkyl(en)restes ist mit mindestens 3 C-Atomen verbunden. Umfasst sind auch Reste R₁, die mehr als eine Verzweigung tragen sowie Reste, die an einem C-Atom 2 Verzweigungen tragen sowie Reste R₁, die verzweigte Verzweigungen tragen. Die weitere(n) Verzweigung(en) kann/können sich sowohl in den Positionen 1, 2, 3 oder 4 zum Sauerstoffatom befinden als auch in den übrigen Positionen des Alky(en)rests befinden.

Geeignete Reste R₁ sind beispielsweise **verzweigte Alkyl(en)reste mit 10 C-Atomen**.

Eine Ausführungsform der Erfindung betrifft Verbindungen der Formel (I), in denen R₁ einen verzweigten Alkyl(en)rest mit 10 C Atomen darstellt, wobei mindestens eine Verzweigung sich in Position 1, 2, 3 oder 4 zum Sauerstoffatom befindet.

Besonders bevorzugt als R₁ sind verzweigte Alkylreste mit 10 C Atomen ausgewählt aus der Gruppe bestehend aus 2-Propylheptyl-, 3-Methyl-2-propyl-hexyl, 4-Methyl-2-propyl-hexyl und 5-Methyl-2-propyl-hexyl-, Trimethylheptyl-, 3,5-Dimethyloctyl-, 2,2-Dimethyloctyl-, 1-Ethyl-octyl-, 2-Ethyl-octyl, 3-Ethyl-octyl-, 4-Ethyl-octyl-, 1-Propyl-heptyl-, 3-Propyl-heptyl-, 4-Propyl-heptyl-.

Geeignete Reste R₁ sind beispielsweise **verzweigte Alkyl(en)reste mit 11 C-Atomen.**

Eine Ausführungsform der Erfindung betrifft Verbindungen der Formel (I), in denen R₁ einen verzweigten Alkyl(en)rest mit 11 C Atomen darstellt, wobei mindestens eine Verzweigung sich in Position 1, 2, 3 oder 4 zum Sauerstoffatom befindet.

Eine Ausführungsform der Erfindung betrifft Verbindungen der Formel (I), in denen R₁ einen Rest darstellt, ausgewählt aus der Gruppe bestehend aus 1-Methyl-decyl-, 2-Methyl-decyl-, 3-Methyl-decyl-, 4-Methyl-decyl-, 1-Ethyl-nonyl-, 2-Ethyl-nonyl-, 3-Ethyl-nonyl-, 4-Ethyl-nonyl-, 1-Propyl-octyl-, 2-Propyl-octyl-, 3-Propyl-octyl-, 4-Propyl-octyl-,

Geeignete Reste R₁ sind beispielsweise **verzweigte Alkyl(en)reste mit 12 C-Atomen.**

Eine Ausführungsform der Erfindung betrifft Verbindungen der Formel (I), in denen R₁ einen verzweigten Alkyl(en)rest mit 12 C Atomen darstellt, wobei mindestens eine Verzweigung sich in Position 1, 2, 3 oder 4 zum Sauerstoffatom befindet.

Eine Ausführungsform der Erfindung betrifft Verbindungen der Formel (I), in denen R₁ einen Rest darstellt, ausgewählt aus der Gruppe bestehend aus 1-Ethyl-decyl-, 2-Ethyl-decyl-, 3-Ethyl-decyl-, 4-Ethyl-decyl-, 1-Butyl-octyl-, 2-Butyl-octyl-, 3-Butyl-octyl-, 4-Butyl-octyl-, 2-Hexyl-hexyl- , 2,5,8-Trimethyl-nonyl; 2,2,4,6,6-Pentamethylheptyl.

Geeignete Reste R₁ sind beispielsweise **verzweigte Alkyl(en)reste mit 13 C-Atomen.**

Eine Ausführungsform der Erfindung betrifft Verbindungen der Formel (I), in denen R₁ einen verzweigten Alkyl(en)rest mit 13 C Atomen darstellt, wobei mindestens eine Verzweigung sich in Position 1, 2, 3 oder 4 zum Sauerstoffatom befindet.

Eine Ausführungsform der Erfindung betrifft Verbindungen der Formel (I), in denen R₁ einen Rest darstellt, ausgewählt aus der Gruppe bestehend aus 1-Methyl-dodecyl-, 2-Methyl-dodecyl-, 3-Methyl-dodecyl-, 4-Methyl-dodecyl-, 1-Ethyl-undecyl-, 2-Ethyl-undecyl-, 3-Ethyl-undecyl-, 4-Ethyl-undecyl-, 1-Propyl-decyl-, 2-Propyl-decyl-, 3-Propyl-decyl-, 4-Propyl-decyl-, Tetramethyl-nonyl.

Geeignete Reste R₁ sind beispielsweise **verzweigte Alkyl(en)reste mit 14 C-Atomen.**

Eine Ausführungsform der Erfindung betrifft Verbindungen der Formel (I), in denen R₁ einen verzweigten Alkyl(en)rest mit 14 C Atomen darstellt, wobei mindestens eine Verzweigung sich in Position 1, 2, 3 oder 4 zum Sauerstoffatom befindet.

Eine Ausführungsform der Erfindung betrifft Verbindungen der Formel (I), in denen R₁ einen Rest darstellt, ausgewählt aus der Gruppe bestehend aus 1-Ethyl-dodecyl-, 2-Ethyl-dodecyl-, 3-Ethyl-dodecyl-, 4-Ethyl-dodecyl-, 1-Butyl-decyl-, 2-Butyl-decyl-, 3-Butyl-decyl-, 4-Butyl-decyl-, 1-Hexyl-octyl-, 2-Hexyl-octyl-, 3-Hexyl-octyl-, 4-Hexyl-octyl-,

Geeignete Reste R₁ sind beispielsweise **verzweigte Alkyl(en)reste mit 15 C-Atomen.**

Eine Ausführungsform der Erfindung betrifft Verbindungen der Formel (I), in denen R₁ einen verzweigten Alkyl(en)rest mit 15 C Atomen darstellt, wobei mindestens eine Verzweigung sich in Position 1, 2, 3 oder 4 zum Sauerstoffatom befindet.

Eine Ausführungsform der Erfindung betrifft Verbindungen der Formel (I), in denen R₁ einen Rest darstellt, ausgewählt aus der Gruppe bestehend aus 1-Methyl-tetradecyl-, 2-Methyl-tetradecyl-, 3-Methyl-tetradecyl-, 4-Methyl-tetradecyl-, 1-Ethyl-tridecyl-, 2-Ethyl-tridecyl-, 3-Ethyl-tridecyl-, 4-Ethyl-tridecyl-, 1-Propyl-dodecyl-, 2-Propyl-dodecyl-, 3-Propyl-dodecyl-, 4-Propyl-dodecyl-, 3,7,11-Trimethyl-dodecyl-.

Geeignete Reste R₁ sind beispielsweise **verzweigte Alkyl(en)reste mit 16 C-Atomen.**

Eine Ausführungsform der Erfindung betrifft Verbindungen der Formel (I), in denen R₁ einen verzweigten Alkyl(en)rest mit 16 C Atomen darstellt, wobei mindestens eine Verzweigung sich in Position 1, 2, 3 oder 4 zum Sauerstoffatom befindet.

Eine Ausführungsform der Erfindung betrifft Verbindungen der Formel (I), in denen R₁ einen Rest darstellt, ausgewählt aus der Gruppe bestehend aus 1-Ethyl-tetradecyl-, 2-Ethyl-tetradecyl-, 3-Ethyl-tetradecyl-, 4-Ethyl-tetradecyl-, 1-Butyl-dodecyl-, 2-Butyl-dodecyl-, 3-Butyl-dodecyl-, 4-Butyl-dodecyl-, 1-Hexyl-decyl-, 2-Hexyl-decyl-, 3-Hexyl-decyl-, 4-Hexyl-decyl-, 2,5,8,11-Tetramethyl-undecyl-.

Geeignete Reste R₁ geeignet sind beispielsweise **verzweigte Alkyl(en)reste mit 17 C-Atomen.** Eine Ausführungsform der Erfindung betrifft Verbindungen der Formel (I), in denen R₁ einen verzweigten Alkyl(en)rest mit 17 C Atomen darstellt, wobei mindestens eine Verzweigung sich in Position 1, 2, 3 oder 4 zum Sauerstoffatom befindet.

Eine Ausführungsform der Erfindung betrifft Verbindungen der Formel (I), in denen R₁ einen Rest darstellt, ausgewählt aus der Gruppe bestehend aus 1-Methyl-hexadecyl-, 2-Methyl-hexadecyl-, 3-Methyl-hexadecyl-, 4-Methyl-hexadecyl-, 1-Ethyl-pentadecyl-, 2-Ethyl-pentadecyl-, 3-Ethyl-pentadecyl-, 4-Ethyl-pentadecyl-, 1-Propyl-tetradecyl-, 2-Propyl-tetradecyl-, 3-Propyl-tetradecyl-, 4-Propyl-tetradecyl-, 1-Butyl-tridecyl-, 2-Butyl-tridecyl-, 3-Butyl-tridecyl-, 4-Butyl-tridecyl-, 1-Pentyl-dodecyl-, 2-Pentyl-dodecyl-, 3-Pentyl-dodecyl-, 4-Pentyl-dodecyl-,

Geeignete Reste R₁ geeignet beispielsweise **verzweigte Alkyl(en)reste mit 18 C-Atomen.** Eine Ausführungsform der Erfindung betrifft Verbindungen der Formel (I), in denen R₁ einen verzweigten Alkyl(en)rest mit 18 C Atomen darstellt, wobei mindestens eine Verzweigung sich in Position 1, 2, 3 oder 4 zum Sauerstoffatom befindet.

Eine Ausführungsform der Erfindung betrifft Verbindungen der Formel (I), in denen R₁ einen Rest darstellt, ausgewählt aus der Gruppe bestehend aus 1-Methyl-heptadecyl-, 2-Methyl-heptadecyl-, 3-Methyl-heptadecyl-, 4-Methyl-heptadecyl-, 1-Ethyl-hexadecyl-, 2-Ethyl-hexadecyl-, 3-Ethyl-hexadecyl-, 4-Ethyl-hexadecyl-, 1-Propyl-pentadecyl-, 2-Propyl-pentadecyl-, 3-Propyl-pentaadecyl-, 4-Propyl-pentadecyl-, 1-Butyl-tetradecyl-, 2-Butyl-tetradecyl-, 3-Butyl-tetradecyl-, 4-Butyl-tetradecyl-, 1-Pentyl-tridecyl-, 2-Pentyl-tridecyl-, 3-Pentyl-tridecyl-, 4-Pentyl-tridecyl-, 1-Hexyl-dodecyl-, 2-Hexyl-dodecyl-, 3-Hexyl-dodecyl-, 4-Hexyl-dodecyl-.

Geeignete Reste R₁ sind beispielsweise **verzweigte Alkyl(en)reste mit 19 C-Atomen.**

Eine Ausführungsform der Erfindung betrifft Verbindungen der Formel (I), in denen R₁ einen verzweigten Alkyl(en)rest mit 19 C Atomen darstellt, wobei mindestens eine Verzweigung sich in Position 1, 2, 3 oder 4 zum Sauerstoffatom befindet.

Eine Ausführungsform der Erfindung betrifft Verbindungen der Formel (I), in denen R₁ einen Rest darstellt, ausgewählt aus der Gruppe bestehend aus
1-Methyl-octadecyl-, 2-Methyl-octadecyl-, 3-Methyl-octadecyl-, 4-Methyl-octadecyl-,
1-Ethyl-heptadecyl-, 2-Ethyl-heptadecyl-, 3-Ethyl-heptadecyl-, 4-Ethyl-heptadecyl-,
1-Propyl-hexadecyl-, 2-Propyl-hexadecyl-, 3-Propyl-hexadecyl-, 4-Propyl-hexadecyl-,
1-Butyl-pentadecyl-, 2-Butyl-pentadecyl-, 3-Butyl-pentadecyl-, 4-Butyl-pentadecyl-,
1-Pentyl-tetradecyl-, 2-Pentyl-tetradecyl-, 3-Pentyl-tetradecyl-, 4-Pentyl-tetradecyl-,
1-Hexyl-tridecyl-, 2-Hexyl-tridecyl-, 3-Hexyl-tridecyl-, 4-Hexyl-tridecyl-,
1-Heptyl-dodecyl-, 2-Heptyl-dodecyl-, 3-Heptyl-dodecyl-, 4-Heptyl-dodecyl-,
1-Octyl-undecyl-,.2-Octyl-undecyl-, 3-Octyl-undecyl-,.4-Octyl-undecyl-,
1-Nonyl-dodecyl-, 2-Nonyl-dodecyl-, 3-Nonyl-dodecyl-, 4-Nonyl-dodecyl-,
1-Hexyl-tridecyl-, 2-Hexyl-tridecyl-, 3-Hexyl-tridecyl-, 4-Hexyl-tridecyl-,

Geeignete Reste R₁ sind beispielsweise **verzweigte Alkyl(en)reste mit 20 C-Atomen.** Eine Ausführungsform der Erfindung betrifft Verbindungen der Formel (I), in denen R₁ einen verzweigten Alkyl(en)rest mit 20 C Atomen darstellt, wobei mindestens eine Verzweigung sich in Position 1, 2, 3 oder 4 zum Sauerstoffatom befindet.

Eine Ausführungsform der Erfindung betrifft Verbindungen der Formel (I), in denen R₁ einen Rest darstellt, ausgewählt aus der Gruppe bestehend aus
1-Methyl-nonadecyl-, 2-Methyl-nonadecyl-, 3-Methyl-nonadecyl-, 4-Methyl-nonadecyl-,
1-Ethyl-octadecyl-, 2-Ethyl-octadecyl-, 3-Ethyl-octadecyl-, 4-Ethyl-octadecyl-,
1-Propyl-heptadecyl-, 2-Propyl-heptadecyl-, 3-Propyl-heptadecyl-, 4-Propyl-heptadecyl-,
1-Butyl-hexadecyl-, 2-Butyl-hexadecyl-, 3-Butyl-hexadecyl-, 4-Butyl-hexadecyl-,
1-Pentyl-pentadecyl-, 2-Pentyl-pentadecyl-, 3-Pentyl-pentadecyl-, 4-Pentyl-pentadecyl-,
1-Hexyl-tetradecyl-, 2-Hexyl-tetradecyl-, 3-Hexyl-tetradecyl-, 4-Hexyl-tetradecyl-,
1-Heptyl-tridecyl-, 2-Heptyl-tridecyl-, 3-Heptyl-tridecyl-, 4-Heptyl-tridecyl-,
1-Octyl-dodecyl-, 2-Octyl-dodecyl-, 3-Octyl-dodecyl-, 4-Octyl-dodecyl-,
1-Nonyl-undecyl-, 2-Nonyl-undecyl-, 3-Nonyl-undecyl-, 4-Nonyl-undecyl-,
1-Decyl-decyl-, 2,5,8,11,13 -Pentamethyl-tridecyl- [= Pentaisobutyl ?]. 3,7,11,15-Tetramethylhexadecyl-.

Geeignete Reste R₁ sind beispielsweise **verzweigte Alkyl(en)reste mit 21 C-Atomen**. Eine Ausführungsform der Erfindung betrifft Verbindungen der Formel (I), in denen R₁ einen verzweigten Alkyl(en)rest mit 21 C Atomen darstellt, wobei mindestens eine Verzweigung sich in Position 1, 2, 3 oder 4 zum Sauerstoffatom befindet.

Eine Ausführungsform der Erfindung betrifft Verbindungen der Formel (I), in denen R₁ einen Rest darstellt, ausgewählt aus der Gruppe bestehend aus
1-Methyl-eicosyl-, 2-Methyl-eicosyl-, 3-Methyl-eicosyl-, 4-Methyl-eicosyl-,
1-Ethyl-nonadecyl-, 2-Ethyl-nonadecyl-, 3-Ethyl-nonadecyl-, 4-Ethyl-nonadecyl-,
1-Propyl-octadecyl-, 2-Propyl-octadecyl-, 3-Propyl-octadecyl-, 4-Propyl-octadecyl-,
1-Butyl-heptdecyl-, 2-Butyl-heptdecyl-, 3-Butyl-heptdecyl-, 4-Butyl-heptdecyl-,
1-Pentyl-hexadecyl-, 2-Pentyl-hexadecyl-, 3-Pentyl-hexadecyl-, 4-Pentyl-hexadecyl-,
1-Hexyl-pentadecyl-, 2-Hexyl-pentadecyl-, 3-Hexyl-pentadecyl-, 4-Hexyl-pentadecyl-,
1-Heptyl-tetradecyl-, 2-Heptyl-tetradecyl-, 3-Heptyl-tetradecyl-, 4-Heptyl-tetradecyl-,
1-Octyl-tridecyl-, 2-Octyl-tridecyl-, 3-Octyl-tridecyl-, 4-Octyl-tridecyl-,
1-Nonyl-dodecyl-, 2-Nonyl-dodecyl-, 3-Nonyl-dodecyl-,
1-Decyl-undecyl-, 2-Decyl-undecyl-,

Geeignete Reste R₁ sind beispielsweise **verzweigte Alkyl(en)reste mit 22 C-Atomen**. Eine Ausführungsform der Erfindung betrifft Verbindungen der Formel (I), in denen R₁ einen verzweigten Alkyl(en)rest mit 21 C Atomen darstellt, wobei mindestens eine Verzweigung sich in Position 1, 2, 3 oder 4 zum Sauerstoffatom befindet.

Eine Ausführungsform der Erfindung betrifft Verbindungen der Formel (I), in denen R₁ einen Rest darstellt, ausgewählt aus der Gruppe bestehend aus
1-Methyl-heneicosyl-, 2-Methyl-heneicosyl-, 3-Methyl-heneicosyl-, 4-Methyl-heneicosyl-,
1-Ethyl-eicosyl-, 2-Ethyl-eicosyl-, 3-Ethyl-eicosyl-, 4-Ethyl-eicosyl-,
1-Propyl-nonadecyl-, 2-Propyl-nonadecyl-, 3-Propyl-nonadecyl-, 4-Propyl-nonadecyl-,
1-Butyl-octadecyl-, 2-Butyl-octadecyl-, 3-Butyl-octadecyl-, 4-Butyl-octadecyl-,
1-Pentyl-heptadecyl-, 2-Pentyl-heptadecyl-,3-Pentyl-heptadecyl-, 4-Pentyl-heptadecyl-,
1-Hexyl-hexadecyl-, 2-Hexyl-hexadecyl-,3-Hexyl-hexadecyl-, 4-Hexyl-hexadecyl-,
1-Heptyl-pentadecyl-, 2-Heptyl-pentadecyl-, 3-Heptyl-pentadecyl-, 4-Heptyl-pentadecyl-,
1-Octyl-tetradecyl-, 2-Octyl-tetradecyl-, 3-Octyl-tetradecyl-, 4-Octyl-tetradecyl-,
1-Nonyl-tridecyl-, 2-Nonyl-tridecyl-, 3-Nonyl-tridecyl-, 4-Nonyl-tridecyl-,
1-Decyl-dodecyl-, 2-Decyl-dodecyl-,

### Rest R₂

Der Rest R₂ stellt einen linearen oder verzweigten Alky(en)rest mit 1 bis 14 C-Atomen dar.

Der Begriff Alkyl(en)rest umfasst gesättigte (= Alkylreste) sowie einfach oder mehrfach ungesättigte Reste (beide als Alkylenreste bezeichnet). Eine bevorzugte Ausführungsform der Erfindung betrifft Verbindungen der Formel (I), in denen R₂ einen gesättigten Rest darstellt.

Der Rest R₂ kann ein verzweigter Alkyl(en)rest sein, d.h. mindestens ein C-Atome des Alkyl(en)restes ist mit mindestens 3 C-Atomen verbunden. Umfasst sind auch Reste R₂, die mehr als eine Verzweigung tragen sowie Reste, die an einem C-Atom 2 Verzweigungen tragen, sowie Reste R₂, die verzweigte Verzweigungen tragen. Die weitere(n) Verzweigung(en) kann/können sich sowohl in den Positionen 1, 2, 3 oder 4 zum Sauerstoffatom befinden als auch in den übrigen Positionen des Alky(en)rests befinden.

Falls R₂ einen verzweigten Alkyl(en)rest darstellt, ist es bevorzugt, dass dieser mindestens eine Verzweigung in Position 1, 2, 3 oder 4 zum Sauerstoffatom trägt. Die Benennung der Positionen der Verzweigungen erfolgt immer ausgehend vom Sauerstoffatom des Ethers, an den der Alkyl(en)rest gebunden ist.

Geeignete Reste R₂ sind beispielsweise Methyl-, Ethyl-.

Geeignete Reste R₂ sind **lineare Alkyl(en)reste mit 3 C Atomen** wie beispielsweise Propyl-; einfach ungesättigte Reste wie 1-Propenyl-, 2-Propenyl-, verzweigte Reste wie beispielsweise Methyl- oder ethyl-.

Geeignete Reste R₂ sind **lineare Alkyl(en)reste mit 4 C Atomen** wie beispielsweise Butyl-; einfach ungesättigte Reste wie beispielsweise But-2-enyl, But-3-enyl, But-1-enyl; Verzweigte Reste R₂ mit 4 C Atomen werden als **i-Butyl-Reste** bezeichnet, Beispiele für i-Butyl-Reste sind 1-Methyl-propyl-, 2-Methyl-propyl-, 1,1-Dimethylethyl-.

Geeignete Reste R₂ sind **lineare Alkyl(en)reste mit 5 C Atomen** wie beispielsweise Pentyl-; einfach ungesättigte Reste wie beispielsweise 1-Pentenyl-, 2-Pentenyl-, 3-Pentenyl-, 4-Pentenyl-. Verzweigte Reste R₂ mit 5 C Atomen werden als **i-Pentyl-Reste** bezeichnet. Beispiele für i-Pentyl-Rest sind 1-Methylbutyl-, 2-Methylbutyl-, 3-Methylbutyl, 1-Ethylpropyl, 1,1-Dimethylpropyl, 1,2-Dimethylpropyl, 2,2-Dimethylpropyl-.

Geeignete Reste R₂ sind **lineare Alkyl(en)reste mit 6 C Atomen,** wie beispielsweise Hexyl-; einfach ungesättigte Reste wie beispielsweise 1-Hexenyl, 2-Hexenyl, 3-Hexenyl, 4-Hexenyl-, 5-Hexenyl-. Verzweigte Reste R₂ mit 6 C Atomen werden als **i-Hexyl Reste** bezeichnet. Beispiele für i-Hexyl Reste sind 1-Methylpentyl-, 2-Methylpentyl, 3-Methylpentyl, 4-Methylpentyl, 1-Ethylbutyl-, 2-Ethylbutyl-, 1,2-Dimethylbutyl-, 1,3-Dimethylbutyl-, 2,2-Dimethylbutyl-, 2,3-Dimethylbutyl-, 3,3-Dimethylbutyl-,

Geeignete Reste R₂ sind **lineare Alkyl(en)reste mit 7 C Atomen,** wie beispielsweise Heptyl-; einfach ungesättigte Reste, wie beispielsweise 1-Hepentyl, 2-Heptenyl, 3-Heptenyl-, 4-Heptenyl-, 5-Heptenyl, 6-Heptenyl-. Verzweigte Reste R₂ mit 7 C Atomen werden als i-**Heptyl Reste** bezeichnet. Beispiele für i-Heptyl Reste sind **Methyl-hexyl-** (1-Methyl-hexyl, 2-Methyl-hexyl, 3-Methyl-hexyl, 4-Methyl-hexyl, 5-Methyl-hexyl-), **Ethyl-pentyl-** (1-Ethyl-pentyl-, 2-Ethyl-pentyl, 3-Ethyl-pentyl oder 4-Ethyl-pentyl-), **Propyl-butyl-** (1-Propyl-butyl-, 2-Propyl-butyl-, 3-Propyl-butyl-), **Dimethyl-pentyl-** (wie beispielsweise 1,1-Dimethyl-pentyl-, 1,2-Dimethyl-pentyl-, 1,3-Dimethyl-pentyl-, 1,4-Dimethyl-pentyl-, 2,2-Dimethyl-pentyl-, 2,3-Dimethyl-pentyl-, 2,4-Dimethyl-pentyl-), **Trimethyl-butyl-** (wie beispielsweise 1,1,2 Trimethyl-butyl, 1,2,3-Trimethylbutyl) oder **Methyl-Ethyl-butyl-** (wie beispielsweise 1-Methyl-2-Ethyl-butyl-). Bevorzugt als i-Heptyl-Rest R₂ ist 1-Ethyl-pentyl.

Geeignete Reste R₂ sind **lineare Alkyl(en)reste mit 8 C Atomen,** wie beispielsweise Octyl-; einfach ungesättigte Reste, wie beispielsweise 1-Octenyl-, 2-Octenyl-, 3-Octenyl-, 4-Octenyl-, 5-Octenyl-, 6- Octenyl-, 7- Octenyl-. Verzweigte Reste R₂ mit 8 C Atomen werden als i-**Octyl Reste** bezeichnet. Beispiele für i-Octyl Reste sind **Methyl-heptyl-** (1-Methyl-heptyl, 2-Methyl-heptyl, 3-Methyl-heptyl, 4-Methyl-heptyl, 5-Methyl-heptyl-, 6-Methyl-heptyl-), **Ethyl-hexyl-** (1-Ethyl-hexyl-, 2-Ethyl-hexyl, 3-Ethyl-hexyl- oder 4-Ethyl-hexyl-), **Propyl-pentyl-** (1-Propyl-pentyl-, 2-Propyl-pentyl-, 3-Propyl-pentyl-), **Butyl-butyl**- (wie beispielsweise 1-Butyl-butyl, 2- Butyl-butyl oder 3-Butyl-butyl-), **Dimethyl-hexyl-** (wie beispielsweise 1,1-Dimethyl-hexyl-, 1,2-Dimethyl-hexyl-, 1,3-Dimethyl-hexyl-, 1,4-Dimethyl-hexyl-, 2,2-Dimethyl-hexyl-, 2,3-Dimethyl-hexyl-, 2,4-Dimethyl-hexyl-), **Trimethyl-pentyl-** (wie beispielsweise 1,1,2 Trimethyl-pentyl, 1,2,3-Trimethylpentyl, 2,4,4-Trimethyl-pentyl) oder **Methyl-Ethyl-pentyl-**(wie beispielsweise 1-Methyl-2-Ethyl-pentyl-) oder **Tetramethyl-butyl** (wie beispielsweise 1,1,3,3 Tetramethyl-butyl). Bevorzugt als i-Octyl-Rest R₂ ist 2,4,4-Trimethyl-pentyl.

Geeignete Reste R₂ sind **lineare Alkyl(en)reste mit 9 C Atomen,** wie beispielsweise Nonyl-; einfach ungesättigte Reste, wie beispielsweise 1-Nonenyl-, 2-Noneyl-, 3-Noneyl-, 4-Noneyl-, 5-Noneyl-, 6-Noneyl-, 7-Noneyl-, 8-Noneyl-. Verzweigte Reste R₂ mit 9 C Atomen werden als i-Nonyl Reste bezeichnet. Beispiele für i-Nonyl Reste sind **Methyl-octyl-** (1-Methyl-octyl-, 2-Methyl-octyl, 3-Methyl-octyl, 4-Methyl-octyl, 5-Methyl-octyl-, 6-Methyl-octyl-, 7-Methyl-octyl-), **Ethyl-heptyl-** (1-Ethyl-heptyl-, 2-Ethyl-heptyl, 3-Ethyl-heptyl, 4-Ethyl-heptyl, 5-Ethyl-heptyl oder 6-Ethyl-heptyl-), **Propyl-hexyl-** (1-Propyl-hexyl-, 2-Propyl-hexyl-, 3-Propyl-hexyl-, 4-Propyl-hexyl-, 5-Propyl-hexyl-), **Butyl-pentyl-** (wie beispielsweise 1-Butyl-pentyl, 2-Butyl-pentyl oder 3-Butyl-pentyl-), Dimethyl-heptyl- (wie beispielsweise 1,1-Dimethyl-heptyl-, 1,2-Dimethyl-heptyl-, 1,3-Dimethyl-heptyl-, 1,4-Dimethyl-heptyl-, 2,2-Dimethyl-heptyl-, 2,3-Dimethyl-heptyl-, 2,4-Dimethyl-heptyl-), **Trimethyl-hexyl-** (wie beispielsweise 1,1,2 Trimethyl-hexyl, 1,2,3-Trimethyl-hexyl, 2-Methyl-4,4-Dimethyl-hexyl, 3,5,5 Trimethyl-hexyl-,) oder **Methyl-Ethyl-hexyl-** (wie beispielsweise 1-Methyl-2-Ethyl-hexyl-). Bevorzugt als i-Nonyl-Rest R₂ sind Trimethyl-hexyl-Reste sowie 3,5, Dimethyl-n-heptyl-.

Geeignete Reste R₂ sind **lineare Alkyl(en)reste mit 10 C Atomen,** wie beispielsweise Decyl-, einfach ungesättigte Reste wie beispielsweise 1-Decenyl-, 2-Decenyl-, 3-Decenyl-, 4-Decenyl sowie **verzweigte Alkyl(en)reste mit 10 C-Atomen,** wie beispielsweise 2-Propylheptyl-, 3-Methyl-2-propyl-hexyl, 4-Methyl-2-propyl-hexyl und 5-Methyl-2-propyl-hexyl-, Trimethylheptyl-, 3,5-Dimethyloctyl-, 2,2-Dimethyloctyl-, 1-Ethyl-octyl-, 2-Ethyl-octyl, 3-Ethyl-octyl-, 4-Ethyl-octyl-, 1-Propyl-heptyl-, 3-Propyl-heptyl-, 4-Propyl-heptyl-.

Geeignete Reste R₂ sind beispielsweise **lineare Alkyl(en)reste mit 11 C-Atomen** wie beispielsweise Undecyl-, einfach ungesättigte Reste wie beispielsweise 1-Undecenyl-, 2-Undecenyl-, 3-Undecenyl-, 4- Undecenyl sowie **verzweigte Alkyl(en)reste mit 11 C-Atomen,** wie beispielsweise 1-Methyl-decyl-, 2-Methyl-decyl-, 3-Methyl-decyl-, 4-Methyl-decyl-, 1-Ethyl-nonyl-, 2-Ethyl-nonyl-, 3-Ethyl-nonyl-, 4-Ethyl-nonyl-, 1-Propyl-octyl-, 2-Propyl-octyl-, 3-Propyl-octyl-, 4-Propyl-octyl-,

Geeignete Reste R₂ sind beispielsweise **lineare Alkyl(en)reste mit 12 C-Atomen** wie beispielsweise Dodecyl-, einfach ungesättigte Reste wie beispielsweise 1-Dodecenyl-, 2-Dodecenyl-, 3-Dodecenyl-, 4-Dodecenyl, 5-Dodecenyl-, sowie **verzweigte Alkyl(en)reste mit 12 C-Atomen.,** wie beispielsweise 1-Ethyl-decyl-, 2-Ethyl-decyl-, 3-Ethyl-decyl-, 4-Ethyl-decyl-, 1-Butyl-octyl-, 2-Butyl-octyl-, 3-Butyl-octyl-, 4-Butyl-octyl-, 2-Hexyl-hexyl-, 2,5,8-Trimethyl-nonyl, 2,2,4,6,6-Pentamethylheptyl-.

Geeignete Reste R₂ sind beispielsweise **lineare Alkyl(en)reste mit 13 C-Atomen** wie beispielsweise Tridecyl-, einfach ungesättigte Reste wie beispielsweise 1-Tridecenyl-, 2-Tridecenyl-, 3-Tridecenyl-, 4-Tridecenyl-, 5-Tridecenyl- sowie **verzweigte Alkyl(en)reste mit** 13 C-Atomen, **wie beispielsweise 1-Methyl-dodecyl-, 2-Methyl-dodecyl-, 3-Methyl-dodecyl-,** 4-Methyl-dodecyl-, 1-Ethyl-undecyl-, 2-Ethyl-undecyl-, 3-Ethyl-undecyl-, 4-Ethyl-undecyl-, 1-Propyl-decyl-, 2-Propyl-decyl-, 3-Propyl-decyl-, 4-Propyl-decyl-, Tetramethyl-nonyl-.

Geeignete Reste R₂ sind beispielsweise **lineare Alkyl(en)reste mit 14 C-Atomen** wie beispielsweise Tetradecyl-; einfach ungesättigte Reste wie beispielsweise 1-Tetradecenyl-, 2-Tetradecenyl-, 3-Tetradecenyl-, 4-Tetradecenyl-, 5-Tetradecenyl-, 6-Tetradecenyl-, sowie **verzweigte Alkyl(en)reste mit 14 C-Atomen,** wie beispielsweise 1-Ethyl-dodecyl-, 2-Ethyl-dodecyl-, 3-Ethyl-dodecyl-, 4-Ethyl-dodecyl-, 1-Butyl-decyl-, 2-Butyl-decyl-, 3-Butyl-decyl-, 4-Butyl-decyl-, 1-Hexyl-octyl-, 2-Hexyl-octyl-, 3-Hexyl-octyl-, 4-Hexyl-octyl-.

### Isomere

Sofern die Reste R₁ oder R₂ ein chirales Kohlenstoffatom aufweisen, sind sowohl die Ether der Racemate als auch die Ether der einzelnen Enantiomere umfasst.

Eine Ausführungsform der Erfindung betrifft Verbindungen der Formel (I),

R₁-O-R₂,

wobei
- R₁ einen verzweigten Alkyl(en)rest **mit 10 C-Atomen** darstellt, wobei mindestens eine Verzweigung sich in Position 1, 2, 3 oder 4 zum Sauerstoffatom befindet
- R₂ einen linearen oder verzweigten Alkyl(en)rest mit 1 bis 13 C Atomen darstellt
- und R₁ und R₂ so gewählt werden, dass die Gesamtzahl der C-Atome in Formel (I) 11 bis 23 beträgt.

Eine bevorzugte Ausführungsform der Erfindung betrifft Verbindungen der Formel (I), in denen R₁ ausgewählt ist aus der Gruppe bestehend aus 2-Propylheptyl-, 3-Methyl-2-propyl-hexyl, 4-Methyl-2-propyl-hexyl und 5-Methyl-2-propyl-hexyl-, Trimethylheptyl-, 3,5-Dimethyloctyl-, 2,2-Dimethyloctyl-, 1-Ethyl-octyl-, 2-Ethyl-octyl, 3-Ethyl-octyl-, 4-Ethyl-octyl-, 1-Propyl-heptyl-, 3-Propyl-heptyl- und 4-Propyl-heptyl-.

Besonders bevorzugt sind Verbindungen der allgemeinen Formel (I), in der R₁ einen 2-Propylheptyl-Rest darstellt. Diese Verbindungen folgen demnach der folgenden Formel:
2-Propyl-heptyl-O-R₂, wobei
   - R₂ einen linearen oder verzweigten Alkyl(en)rest mit 1 bis 13 C Atomen darstellt
   - und R₁ und R₂ so gewählt werden, dass die Gesamtzahl der C-Atome in Formel (I) 11 bis 23 beträgt.

Eine bevorzugte Verbindung ist 2-Propyl-heptyl-methylether, die Verbindung der folgenden Formel:

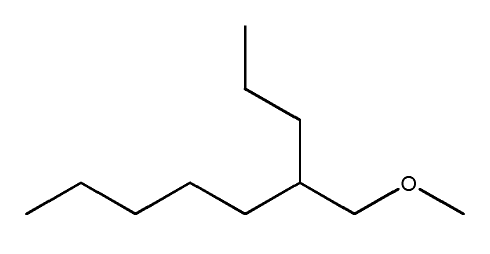

Eine bevorzugte Verbindung ist 2-Propyl-heptyl-ethylether, die Verbindung der folgenden Formel:

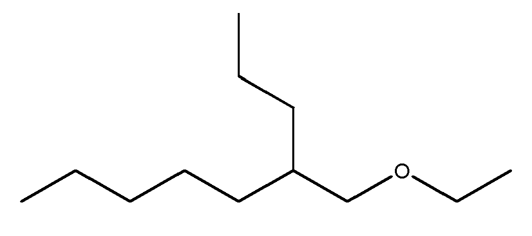

Eine bevorzugte Verbindung ist 2-Propyl-heptyl-butylether, die Verbindung der folgenden Formel:

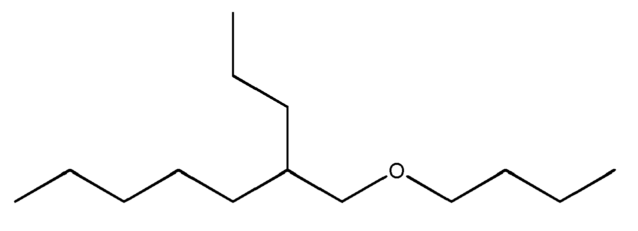

Eine bevorzugte Ausführungsform der Erfindung betrifft Verbindungen der Formel (I), in denen R₁ und R₂ einen 2-Propylheptyl-Rest darstellt. Diese Verbindung folgt demnach der folgenden Formel (= Di(2-Propylheptyl)ether):

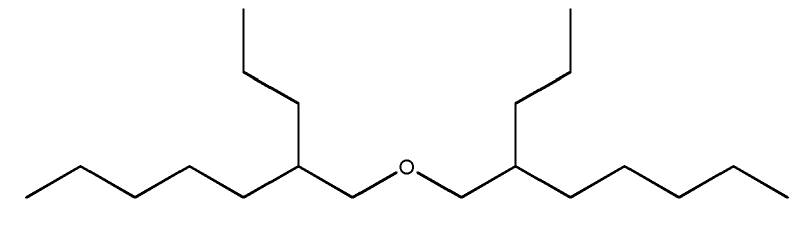

Die folgende Tabelle führt zusammen mit den bereits genannten Verbindungen weitere bevorzugte Verbindungen auf

| Nr. | R₁= | R₂ = |
|---|---|---|
| | 2-Propyl-heptyl- | Methyl- |
| | 2-Propyl-heptyl- | Ethyl- |
| | 2-Propyl-heptyl- | n-Propyl- |
| | 2-Propyl-heptyl- | 2-Methyl-ethyl- |
| | 2-Propyl-heptyl- | 1-Propenyl- |
| | 2-Propyl-heptyl- | 2-Propenyl- |
| | | |
| | 2-Propyl-heptyl- | n-Butyl- |
| | 2-Propyl-heptyl | But-1-enyl- |
| | 2-Propyl-heptyl | But-2-enyl- |
| | 2-Propyl-heptyl | But-3-enyl- |
| | 2-Propyl-heptyl | 1-Methyl-propyl- |
| | 2-Propyl-heptyl | 2-Methyl-propyl- |
| | 2-Propyl-heptyl | 1,1-Dimethylethyl- |
| | | |
| | 2-Propyl-heptyl- | n-Pentyl- |
| | 2-Propyl-heptyl- | 1-Pentenyl- |
| | 2-Propyl-heptyl- | 2-Pentenyl- |
| | 2-Propyl-heptyl- | 3-Pentenyl- |
| | | 4-Pentenyl- |
| | 2-Propyl-heptyl- | 1-Methylbutyl- |
| | 2-Propyl-heptyl- | 2-Methylbutyl- |
| | 2-Propyl-heptyl- | 3-Methylbutyl- |
| | 2-Propyl-heptyl- | 1-Ethylpropyl- |
| | 2-Propyl-heptyl- | 1,1-Dimethylpropyl- |
| | 2-Propyl-heptyl- | 1,2-Dimethylpropyl- |
| | 2-Propyl-heptyl- | 2,2-Dimethylpropyl- |
| | | |
| | 2-Propyl-heptyl- | n-Hexyl- |
| | 2-Propyl-heptyl- | 1-Hexenyl- |
| | 2-Propyl-heptyl- | 2-Hexenyl- |
| | 2-Propyl-heptyl- | 3-Hexenyl- |
| | 2-Propyl-heptyl- | 4-Hexenyl- |
| | 2-Propyl-heptyl- | 5-Hexenyl- |
| | 2-Propyl-heptyl- | 1-Methylpentyl- |
| | 2-Propyl-heptyl- | 2-Methylpentyl- |
| | 2-Propyl-heptyl- | 3-Methylpentyl- |
| | 2-Propyl-heptyl- | 4-Methylpentyl- |
| | 2-Propyl-heptyl- | 1-Ethylbutyl- |
| | 2-Propyl-heptyl- | 2-Ethylbutyl- |
| | 2-Propyl-heptyl- | 1,2-Dimethylbutyl- |
| | 2-Propyl-heptyl- | 1,3-Dimethylbutyl- |
| | 2-Propyl-heptyl- | 2,2-Dimethylbutyl- |
| | 2-Propyl-heptyl | 2,3-Dimethylbutyl- |
| | 2-Propyl-heptyl- | 3,3-Dimethylbutyl-, |
| | | |
| | 2-Propyl-heptyl- | n-Heptyl- |
| | 2-Propyl-heptyl- | 1-Hepentyl- |
| | 2-Propyl-heptyl- | 2-Heptenyl- |
| | | |
| | 2-Propyl-heptyl- | 3-Heptenyl- |
| | 2-Propyl-heptyl- | 4-Heptenyl- |
| | 2-Propyl-heptyl- | 5-Heptenyl- |
| | 2-Propyl-heptyl- | 6-Heptenyl- |
| | 2-Propyl-heptyl- | 1-Methyl-hexyl- |
| | 2-Propyl-heptyl- | 2-Methyl-hexyl- |
| | 2-Propyl-heptyl- | 3-Methyl-hexyl- |
| | 2-Propyl-heptyl- | 4-Methyl-hexyl- |
| | 2-Propyl-heptyl- | 5-Methyl-hexyl- |
| | 2-Propyl-heptyl- | 1-Ethyl-pentyl-, |
| | 2-Propyl-heptyl- | 2-Ethyl-pentyl-, |
| | 2-Propyl-heptyl- | 3-Ethyl-pentyl- |
| | 2-Propyl-heptyl- | 4-Ethyl-pentyl- |
| | 2-Propyl-heptyl- | 1-Propyl-butyl- |
| | 2-Propyl-heptyl- | 2-Propyl-butyl-, |
| | 2-Propyl-heptyl- | 3-Propyl-butyl- |
| | 2-Propyl-heptyl- | 1,1-Dimethyl-pentyl- |
| | 2-Propyl-heptyl- | 1,2-Dimethyl-pentyl- |
| | 2-Propyl-heptyl- | 1,3-Dimethyl-pentyl- |
| | 2-Propyl-heptyl- | 1,4-Dimethyl-pentyl- |
| | 2-Propyl-heptyl- | 2,2-Dimethyl-pentyl- |
| | 2-Propyl-heptyl- | 2,3-Dimethyl-pentyl- |
| | 2-Propyl-heptyl- | 2,4-Dimethyl-pentyl- |
| | 2-Propyl-heptyl- | 1,1,2 Trimethyl-butyl- |
| | 2-Propyl-heptyl- | 1,2,3-Trimethylbutyl- |
| | 2-Propyl-heptyl- | 1-Methyl-2-Ethyl-butyl- |
| | | |
| | 2-Propyl-heptyl- | n-Octyl- |
| | 2-Propyl-heptyl- | 1-Octenyl- |
| | 2-Propyl-heptyl- | 2-Octenyl- |
| | 2-Propyl-heptyl- | 3-Octenyl- |
| | 2-Propyl-heptyl- | 4-Octenyl- |
| | 2-Propyl-heptyl- | 5-Octenyl- |
| | 2-Propyl-heptyl- | 1-Methyl-heptyl- |
| | 2-Propyl-heptyl- | 2-Methyl-heptyl- |
| | 2-Propyl-heptyl- | 3-Methyl-heptyl- |
| | 2-Propyl-heptyl- | 4-Methyl-heptyl- |
| | 2-Propyl-heptyl- | 5-Methyl-heptyl- |
| | 2-Propyl-heptyl- | 6-Methyl-heptyl- |
| | 2-Propyl-heptyl- | 1-Ethyl-hexyl- |
| | 2-Propyl-heptyl- | 2-Ethyl-hexyl- |
| | 2-Propyl-heptyl- | 3-Ethyl-hexyl- |
| | 2-Propyl-heptyl- | 4-Ethyl-hexyl- |
| | 2-Propyl-heptyl- | 1-Propyl-pentyl- |
| | 2-Propyl-heptyl- | 2-Propyl-pentyl- |
| | 2-Propyl-heptyl- | 3-Propyl-pentyl- |
| | 2-Propyl-heptyl- | 1-Butyl-butyl- |
| | 2-Propyl-heptyl- | 2-Butyl-butyl- |
| | 2-Propyl-heptyl- | 3-Butyl-butyl- |
| | 2-Propyl-heptyl- | 1,1-Dimethyl-hexyl- |
| | 2-Propyl-heptyl- | 1,2-Dimethyl-hexyl- |
| | 2-Propyl-heptyl- | 1,3-Dimethyl-hexyl- |
| | 2-Propyl-heptyl- | 1,4-Dimethyl-hexyl- |
| | 2-Propyl-heptyl- | 2,2-Dimethyl-hexyl- |
| | 2-Propyl-heptyl- | 2,3-Dimethyl-hexyl- |
| | 2-Propyl-heptyl- | 2,4-Dimethyl-hexyl- |
| | 2-Propyl-heptyl- | 1,1,2 Trimethyl-pentyl- |
| | 2-Propyl-heptyl- | 1,2,3-Trimethylpentyl- |
| | 2-Propyl-heptyl- | 2,4,4-Trimethyl-pentyl- |
| | 2-Propyl-heptyl- | 1-Methyl-2-Ethyl-pentyl- |
| | 2-Propyl-heptyl- | 1,1,3,3 Tetramethyl-butyl- |
| | | |
| | 2-Propyl-heptyl- | n-Nonyl- |
| | 2-Propyl-heptyl- | 1-Nonenyl- |
| | 2-Propyl-heptyl- | 2-Noneyl- |
| | 2-Propyl-heptyl- | 3-Noneyl- |
| | 2-Propyl-heptyl- | 4-Noneyl- |
| | 2-Propyl-heptyl- | 5-Noneyl- |
| | 2-Propyl-heptyl- | 6-Noneyl- |
| | 2-Propyl-heptyl- | 7-Noneyl- |
| | 2-Propyl-heptyl- | 8-Noneyl- |
| | 2-Propyl-heptyl- | 1-Methyl-octyl- |
| | 2-Propyl-heptyl- | 2-Methyl-octyl- |
| | 2-Propyl-heptyl- | 3-Methyl-octyl- |
| | 2-Propyl-heptyl- | 4-Methyl-octyl- |
| | 2-Propyl-heptyl- | 5-Methyl-octyl- |
| | 2-Propyl-heptyl- | 6-Methyl-octyl- |
| | 2-Propyl-heptyl- | 7-Methyl-octyl- |
| | 2-Propyl-heptyl- | 1-Ethyl-heptyl- |
| | 2-Propyl-heptyl- | 2-Ethyl-heptyl- |
| | 2-Propyl-heptyl- | 3-Ethyl-heptyl- |
| | 2-Propyl-heptyl- | 4-Ethyl-heptyl- |
| | 2-Propyl-heptyl- | 5-Ethyl-heptyl- |
| | 2-Propyl-heptyl- | 6-Ethyl-heptyl- |
| | 2-Propyl-heptyl- | 1-Propyl-hexyl- |
| | 2-Propyl-heptyl- | 2-Propyl-hexyl- |
| | 2-Propyl-heptyl- | 3-Propyl-hexyl- |
| | 2-Propyl-heptyl- | 4-Propyl-hexyl- |
| | 2-Propyl-heptyl- | 5-Propyl-hexyl- |
| | 2-Propyl-heptyl- | 1-Butyl-pentyl- |
| | 2-Propyl-heptyl- | 2-Butyl-pentyl- |
| | 2-Propyl-heptyl- | 3-Butyl-pentyl- |
| | 2-Propyl-heptyl- | 1,1-Dimethyl-heptyl- |
| | 2-Propyl-heptyl- | 1,2-Dimethyl-heptyl- |
| | 2-Propyl-heptyl- | 1,3-Dimethyl-heptyl- |
| | 2-Propyl-heptyl- | 1,4-Dimethyl-heptyl- |
| | 2-Propyl-heptyl- | 2,2-Dimethyl-heptyl- |
| | 2-Propyl-heptyl- | 2,3-Dimethyl-heptyl- |
| | 2-Propyl-heptyl- | 2,4-Dimethyl-heptyl- |
| | 2-Propyl-heptyl- | 1,1,2 Trimethyl-hexyl- |
| | 2-Propyl-heptyl- | 1,2,3-Trimethyl-hexyl- |
| | 2-Propyl-heptyl- | 2-Methyl-4,4-Dimethyl-hexyl- |
| | 2-Propyl-heptyl- | 3,5,5 Trimethyl-hexyl- |
| | 2-Propyl-heptyl- | 1-Methyl-2-Ethyl-hexyl- |
| | | |
| | 2-Propyl-heptyl- | Decyl- |
| | 2-Propyl-heptyl- | 1-Decenyl- |
| | 2-Propyl-heptyl- | 2-Decenyl- |
| | 2-Propyl-heptyl- | 3-Decenyl- |
| | 2-Propyl-heptyl- | 4- Decenyl- |
| | 2-Propyl-heptyl- | 1-Propyl-heptyl-, |
| | 2-Propyl-heptyl- | 2-Propylheptyl- |
| | 2-Propyl-heptyl- | 3-Propyl-heptyl- |
| | 2-Propyl-heptyl- | 4-Propyl-heptyl-. |
| | 2-Propyl-heptyl- | 1-Ethyl-octyl- |
| | 2-Propyl-heptyl- | 2-Ethyl-octyl- |
| | 2-Propyl-heptyl- | 3-Ethyl-octyl- |
| | 2-Propyl-heptyl- | 4-Ethyl-octyl- |
| | 2-Propyl-heptyl- | 3-Methyl-2-propyl-hexyl- |
| | 2-Propyl-heptyl- | 4-Methyl-2-propyl-hexyl- |
| | 2-Propyl-heptyl- | 5-Methyl-2-propyl-hexyl- |
| | 2-Propyl-heptyl- | Trimethylheptyl-, |
| | 2-Propyl-heptyl- | 3,5-Dimethyloctyl- |
| | 2-Propyl-heptyl- | 2,2-Dimethyloctyl- |
| | | |
| | 2-Propyl-heptyl- | Undecyl- |
| | 2-Propyl-heptyl- | 1-Undecenyl- |
| | 2-Propyl-heptyl- | 2-Undecenyl- |
| | 2-Propyl-heptyl- | 3-Undecenyl- |
| | 2-Propyl-heptyl- | 4- Undecenyl- |
| | 2-Propyl-heptyl- | 1-Methyl-decyl- |
| | 2-Propyl-heptyl- | 2-Methyl-decyl- |
| | 2-Propyl-heptyl- | 3-Methyl-decyl- |
| | 2-Propyl-heptyl- | 4-Methyl-decyl- |
| | 2-Propyl-heptyl- | 1-Ethyl-nonyl- |
| | 2-Propyl-heptyl- | 2-Ethyl-nonyl- |
| | 2-Propyl-heptyl- | 3-Ethyl-nonyl- |
| | 2-Propyl-heptyl- | 4-Ethyl-nonyl- |
| | 2-Propyl-heptyl- | 1-Propyl-octyl- |
| | 2-Propyl-heptyl- | 2-Propyl-octyl- |
| | 2-Propyl-heptyl- | 3-Propyl-octyl- |
| | 2-Propyl-heptyl- | 4-Propyl-octyl- |
| | | |
| | 2-Propyl-heptyl- | Dodecyl- |
| | 2-Propyl-heptyl- | 1-Dodecenyl- |
| | 2-Propyl-heptyl- | 2-Dodecenyl- |
| | 2-Propyl-heptyl- | 3-Dodecenyl- |
| | 2-Propyl-heptyl- | 4-Dodecenyl- |
| | 2-Propyl-heptyl- | 5-Dodencenyl- |
| | 2-Propyl-heptyl- | 1-Ethyl-decyl- |
| | 2-Propyl-heptyl- | 2-Ethyl-decyl- |
| | 2-Propyl-heptyl- | 3-Ethyl-decyl- |
| | 2-Propyl-heptyl- | 4-Ethyl-decyl- |
| | 2-Propyl-heptyl- | 1-Butyl-octyl- |
| | 2-Propyl-heptyl- | 2-Butyl-octyl- |
| | 2-Propyl-heptyl- | 3-Butyl-octyl- |
| | 2-Propyl-heptyl- | 4-Butyl-octyl- |
| | 2-Propyl-heptyl- | 2-Hexyl-hexyl- |
| | 2-Propyl-heptyl- | 2,5,8-Trimethyl-nonyl |
| | 2-Propyl-heptyl- | 2,2,4,6,6-Pentamethylheptyl |
| | | |
| | 2-Propyl-heptyl- | Tridecyl-, |
| | 2-Propyl-heptyl- | 1-Tridecenyl- |
| | 2-Propyl-heptyl- | 2-Tridecenyl- |
| | 2-Propyl-heptyl- | 3-Tridecenyl- |
| | 2-Propyl-heptyl- | 4-Tridecenyl- |
| | 2-Propyl-heptyl- | 5-Tridecenyl- |
| | 2-Propyl-heptyl- | 1-Methyl-dodecyl- |
| | 2-Propyl-heptyl- | 2-Methyl-dodecyl- |
| | 2-Propyl-heptyl- | 3-Methyl-dodecyl- |
| | 2-Propyl-heptyl- | 4-Methyl-dodecyl- |
| | 2-Propyl-heptyl- | 1-Ethyl-undecyl- |
| | 2-Propyl-heptyl- | 2-Ethyl-undecyl- |
| | 2-Propyl-heptyl- | 3-Ethyl-undecyl- |
| | 2-Propyl-heptyl- | 4-Ethyl-undecyl- |
| | 2-Propyl-heptyl- | 1-Propyl-decyl- |
| | 2-Propyl-heptyl- | 2-Propyl-decyl- |
| | 2-Propyl-heptyl- | 3-Propyl-decyl- |
| | 2-Propyl-heptyl- | 4-Propyl-decyl- |
| | 2-Propyl-heptyl- | Tetramethyl-nonyl [= Isotridecyl-] |
| | | |
| | 2-Propyl-heptyl- | Tetradecyl- |
| | 2-Propyl-heptyl- | 1-Tetradecenyl- |
| | 2-Propyl-heptyl- | 2-Tetradecenyl- |
| | 2-Propyl-heptyl- | 3-Tetradecenyl- |
| | 2-Propyl-heptyl- | 4-Tetradecenyl- |
| | 2-Propyl-heptyl- | 5-Tetradecenyl- |
| | 2-Propyl-heptyl- | 6-Tetradecenyl- |
| | 2-Propyl-heptyl- | 1-Ethyl-dodecyl- |
| | 2-Propyl-heptyl- | 2-Ethyl-dodecyl- |
| | 2-Propyl-heptyl- | 3-Ethyl-dodecyl- |
| | 2-Propyl-heptyl- | 4-Ethyl-dodecyl- |
| | 2-Propyl-heptyl- | 1-Butyl-decyl- |
| | 2-Propyl-heptyl- | 2-Butyl-decyl-, |
| | 2-Propyl-heptyl- | 3-Butyl-decyl-, |
| | 2-Propyl-heptyl- | 4-Butyl-decyl-, |
| | 2-Propyl-heptyl- | 1-Hexyl-octyl-. |
| | 2-Propyl-heptyl- | 2-Hexyl-octyl- |
| | 2-Propyl-heptyl- | 3-Hexyl-octyl- |
| | 2-Propyl-heptyl- | 4-Hexyl-octyl- |

### Kosmetische/pharmazeutische Zubereitungen

Überraschenderweise sind die erfindungsgemäßen Verbindungen besonders gut für kosmetische und/oder pharmazeutische Zubereitungen geeignet, insbesondere für Zubereitungen, bei denen es auf ein "leichtes" Hautgefühl ankommt. Die Verbindungen lassen sich sehr gut in verschiedene Formulierungen einarbeiten. Sie weisen ein sensorisches Profil auf, das demjenigen von flüchtigen Silikonen, wie beispielsweise Cyclomethicone, vergleichbar ist. Sie zeigen gegenüber bekannten Dialkylethern eine verbesserte Hautverträglichkeit.

Erfindungsgemäß kann eine einzelne Verbindung gemäß Anspruch 1 oder ein beliebiges Gemisch verschiedener Verbindungen gemäß Anspruch 1 eingesetzt werden.

Ein Gegenstand der Erfindung betrifft daher die Verwendung einer oder mehrerer Verbindungen der allgemeinen Formel (I)

R₁-O-R₂,

wobei
- R₁ einen verzweigten Alkyl(en)rest mit 10 bis 22 C-Atomen darstellt, wobei mindestens eine Verzweigung sich in Position 1, 2, 3 oder 4 zum Sauerstoffatom befindet
- R₂ einen linearen oder verzweigten Alkyl(en)rest mit 1 bis 13 C Atomen darstellt
- und R₁ und R₂ so gewählt werden, dass die Gesamtzahl der C-Atome in Formel (I) 11 bis 23 beträgt.
zur Herstellung von oder in kosmetischen und/oder pharmazeutischen Zubereitungen, insbesondere als Ölkörper und/oder als Lösevermittler und/oder als Dispergiermittel in kosmetischen und/oder pharmazeutischen Zubereitungen.

Ein weiterer Gegenstand der Erfindung ist insbesondere die Verwendung der Verbindungen gemäß Formel (I) in kosmetischen und/oder pharmazeutischen Zubereitungen zur Benetzung oder Imprägnierung oder Beschichtung von Gebrauchs- und/oder Hygienetüchern, die zur Körperreinigung und/oder zur Körperpflege eingesetzt werden.

Ein weiterer Gegenstand der vorliegenden Erfindung sind kosmetische und/oder pharmazeutische Zubereitungen enthaltend enthaltend in einem kosmetisch und/oder pharmazeutisch geeigneten Träger 0,1 bis 95 Gew.-% einer oder mehrerer Verbindungen nach einem der Ansprüche 1 bis 5.

Ein weiterer Gegenstand der vorliegenden Erfindung sind kosmetische und/oder pharmazeutische Zubereitungen enthaltend
(a) wenigstens eine Verbindung gemäß Anspruch 1 bis 5
(b) wenigstens eine grenzflächen-aktive Substanz (b-1) und/oder Wachskomponente (b-2) und/oder Polymer (b-3) und/oder einen weiteren Ölkörper (b-4)

Ein weiterer Gegenstand der vorliegenden Erfindung sind kosmetische und/oder pharmazeutische Zubereitungen enthaltend
(a) wenigstens einen Verbindung gemäß Anspruch 1 bis 5
(d) wenigstens einen UV-Lichtschutzfilter

Bevorzugt sind kosmetische und/oder pharmazeutische Zubereitungen enthaltend
(a) wenigstens eine Verbindung der allgemeinen Formel (I)

   R₁-O-R₂,

   wobei
   - R₁ einen verzweigten Alkyl(en)rest **mit 10 C-Atomen** darstellt, wobei mindestens eine Verzweigung sich in Position 1, 2, 3 oder 4 zum Sauerstoffatom befindet
   - R₂ einen linearen oder verzweigten Alkyl(en)rest mit 1 bis 13 C Atomen darstellt
   - und R₁ und R₂ so gewählt werden, dass die Gesamtzahl der C-Atome in Formel (I) 11 bis 23 beträgt.
(b) wenigstens eine grenzflächen-aktive Substanz (b-1) und/oder Wachskomponente (b-2) und/oder Polymer (b-3) und/oder einen weiteren Ölkörper (b-4)

Eine bevorzugte Ausführungsform der Erfindung betrifft kosmetische und/oder pharmazeutische Zubereitungen enthaltend
(a) wenigstens eine Verbindung der allgemeinen Formel (I)

   R₁-O-R₂,

   wobei
   - R₁ einen verzweigten Alkyl(en)rest darstellt, ausgewählt aus der Gruppe bestehend aus 2-Propylheptyl-, 3-Methyl-2-propyl-hexyl, 4-Methyl-2-propyl-hexyl und 5-Methyl-2-propyl-hexyl-, Trimethylheptyl-, 3,5-Dimethyloctyl-, 2,2-Dimethyloctyl-, 1-Ethyl-octyl-, 2-Ethyl-octyl, 3-Ethyl-octyl-, 4-Ethyl-octyl-, 1-Propyl-heptyl-, 3-Propyl-heptyl- und 4-Propyl-heptyl-.
   - R₂ einen linearen oder verzweigten Alkyl(en)rest mit 1 bis 13 C Atomen darstellt
   - und R₁ und R₂ so gewählt werden, dass die Gesamtzahl der C-Atome in Formel (I) 11 bis 23 beträgt.
(b) wenigstens einen Emulgator (b-1) und/oder Tensid (b-2) und/oder Wachskomponente (b-3) und/oder Polymer (b-4) und/oder einen weiteren Ölkörper (b-5)

Besonders bevorzugt sind kosmetische und/oder pharmazeutische Zubereitungen enthaltend
(a) wenigstens eine Verbindung der allgemeinen Formel (I)

   R₁-O-R₂,

   wobei
   - R₁ einen 2-Propylheptyl-Rest darstellt
   - R₂ einen linearen oder verzweigten Alkyl(en)rest mit 1 bis 13 C Atomen darstellt
   - und R₁ und R₂ so gewählt werden, dass die Gesamtzahl der C-Atome in Formel (I) 11 bis 23 beträgt.
(b) wenigstens einen Emulgator (b-1) und/oder Tensid (b-2) und/oder Wachskomponente (b-3) und/oder Polymer (b-4) und/oder einen weiteren Ölkörper (b-5)

Vorzugsweise enthalten die erfindungsgemäßen Zubereitungen 0,1 bis 95, insbesondere 0,2 bis 80 Gew.-%, insbesondere 0,5 bis 70, vorzugsweise 0,75 bis 60 Gew.-%, insbesondere 1 bis 50 Gew.-%, bevorzugt 1 - 40 Gew.-% wenigstens einer Verbindung gemäß Formel (I).

Ein weiterer Gegenstand der Erfindung sind Zubereitungen kosmetische und/oder pharmazeutische Zubereitungen enthaltend
a) 0,1 - 95 Gew.-%, insbesondere 0,2 bis 80 Gew.-%, insbesondere 0,1 bis 70, bevorzugt 0,1 bis 60, insbesondere 0,1 bis 50 Gew.-%, bevorzugt 0,1 - 40 Gew.-% wenigstens einer Verbindung gemäß Anspruch 1,
b) 0,1 - 20 Gew.-% grenzflächen-aktive Substanz (b-1) und/oder Wachskomponente (b-2) und/oder Polymer (b-3), 0,1 - 40 Gew.-% weiterer Ölkörper (b-4) und
c) 0 - 98 Gew.-% Wasser.

Die erfindungsgemäßen Zubereitungen enthalten vorzugsweise mindestens 0,1, insbesondere mindestens 0,5, insbesondere mindestens 0,75, bevorzugt mindestens 1, bevorzugt mindestens 5 Gew.-% eines oder mehrerer Verbindungen gemäß Anspruch 1.

Alle Gew.-% Angaben beziehen sich auf Gew.-% bezogen auf die kosmetische und/oder pharmazeutische Zubereitung.

Die erfindungsgemäßen Zubereitungen, sowie die erfindungsgemäßen Verbindungen der allgemeinen Formel (I) eignen sich als Basis in allen kosmetischen Mittel zur Körperpflege und -reinigung wie z.B. Körperöl, Babyöl, Körpermilch, Cremes, Lotionen, sprühbare Emulsionen, Sonnenschutzmittel, Antitranspirantien, Flüssig- und Stückseifen etc. eingearbeitet werden. Sie lassen sich auch in tensidhaltigen Zubereitungen wie z.B. Schaum- und Duschbädern, Haarshampoos und Pflegespülungen einsetzen. Sie lassen sich als Pflegekomponente auf Tissues, Papiere, Wipes, Vlies-Produkte, Schwämme, Puffs, Pflaster und Bandagen applizieren, die ihren Einsatz im Bereich der Hygiene und Pflege finden (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte, Selbstbräunungs Wipes). Sie lassen sich u.a. auch in Zubereitungen zur Haarpflege, Haarreinigung oder Haarfärbung einsetzen. Sie lassen sich weiterhin in Zubereitungen der dekorativen Kosmetik, wie beispielsweise Lippenstiften, lipgloss, Make-up, Foundations, Puder, Lidschatten, Maskara und ähnlichem einsetzen

Je nach Applikationszweck enthalten die kosmetischen Formulierungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Füllstoffe, Hydrotrope, Solubilisatoren, Konservierungsmittel, Parfümöle, Farbstoffe etc., die nachstehend exemplarisch aufgelistet sind.

### Grenzflächen-aktive Substanz b-1)

In einer Ausführungsform der Erfindung enthalten die erfindungsgemäßen Zubereitungen mindestens eine grenzflächen-aktive Substanz. Die erfindungsgemäßen Zubereitungen enthalten den/die grenzflächen-aktiven Substanz(en) in einer Menge von 0 bis 80 Gew.-% , insbesonderen 0 bis 40 Gew.-%, vorzugsweise 0,1 bis 20 Gew.-% vorzugsweise 0,1 bis 15 Gew.-% und insbesondere 0,1 bis 10 Gew.-% bezogen auf das Gesamtgewicht der Zubereitung.

Als grenzflächen-aktive Substanz eignet sich prinzipiell jede Substanz, welche die Oberflächenspannung zwischen der wässrigen und der nicht-wässrigen Phase erniedrigt. Grenzflächen-aktive Substanzen umfassen Emulgatoren und Tenside.

In einer Ausführungsform der Erfindung enthält die erfindungsgemäße Zubereitung mehr als eine grenzflächen-aktive Substanz. Der Fachmann setzt in Abhängigkeit der übrigen Komponenten übliche Systeme (wie z.B. Emulgator und Co-Emulgator) ein.

Ein geeigneter Emulgator ist prinzipiell jede grenzflächen-aktive Substanz, insbesondere jedoch Substanzen mit einem HLB-Wert von 1 bis 20 nach der Griffin Skala. Jedem Emulgator wird ein so genannter HLB-Wert (eine dimensionslose Zahl zwischen 1 und 20, Griffin Skala) zugeschrieben, der angibt, ob eine bevorzugte Wasser- oder Öllöslichkeit vorliegt. Zahlen unter 9 kennzeichnen bevorzugt öllösliche, hydrophobe Emulgatoren, Zahlen über 11 wasserlösliche, hydrophile Emulgatoren. Der HLB-Wert sagt etwas über das Gleichgewicht der Größe und Stärke der hydrophilen und der lipophilen Gruppen eines Emulgators aus. Die Griffin Skala ist beschrieben in WC Griffin, J. Soc. Cosmet. Chem. 1 (1949) 311**;** WC Griffin, J. Soc. Cosmet. Chem. 5 ( 1954) 249**.**

Der HLB-Wert eines Emulgators lässt sich auch aus Inkrementen errechnen, wobei die HLB-Inkremente für die verschiedenen hydrophilen und hydrophoben Gruppen, aus denen sich ein Molekül zusammensetzt, Tabellenwerken (z. B. H. P. Fiedler, Lexikon der Hilfsstoffe für Pharmazie, Kosmetik und angrenzende Gebiete, Editio Cantor Verlag, Aulendorf, 4. Aufl. 1996**)** oder den Herstellerangaben entnommen werden kann. Die Löslichkeit des Emulgators in den beiden Phasen bestimmt praktisch den Emulsionstyp. Ist der Emulgator besser in Wasser löslich erhält man eine O/W-Emulsion. Hat der Emulgator hingegen eine bessere Löslichkeit in der Ölphase entsteht unter sonst gleichen Herstellungsbedingungen eine W/O-Emulsion.

### Nicht-ionische Emulgatoren

Zur Gruppe der nicht-ionischen Emulgatoren gehören beispielsweise:
(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) C₁₂-C₁₈-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglyceryl-4-Laurate, Polyglycerindiisostearat oder Polyglycerindimerat. Ebenfalls geeignet sind Gemische von Verbindungen aus mehreren dieser Substanzklassen, wie z.B.Polyglyceryl-4 Düsostearate/Polyhydroxystearate/Sebacate
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter C₆-C₂₂-Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester, sowie Sucrose Polystearate (kommerziell erhältlich als Emulgade® SUCRO, Cognis GmbH).
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. C_{12/18}-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls^{®} PGPH" (W/O-Emulgator) oder "Eumulgin^{®} VL 75" (Abmischung mit Coco Glucosides im Gewichtsverhältnis 1:1, O/W-Emulgator) oder Dehymuls^{®} SBL (W/O-Emulgator) von der Cognis Deutschland GmbH vertrieben werden. In diesem Zusammenhang sei insbesondere auf das Europäische Patent EP 766 661B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in Kirk-Othmer, "Encyclopedia of Chemical Technology", 3. Aufl., 1979, Band 8, Seite 913**,** aufgelistet. Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von C₄-C₆-Polyolen, wie beispielsweise Partialester des Pentaerythrits oder Zuckerestern, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitan-dihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitan-trimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nicht-ionischer O/W-Emulgatoren (HLB-Wert: 8- 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12, Ceteareth-20 und PEG-20 Stearat. Als Solubilisatoren bevorzugt geeignet sind Eumulgin^{®} HRE 40 (INCI: PEG-40 Hydrogenated Castor Oil), Eumulgin^{®} HRE 60 (INCI: PEG-60 Hydrogenated Castor Oil), Eumulgin^{®} L (INCl: PPG-1-PEG-9 Laurylglycolether), sowie Eumulgin^{®} SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. C₈-C₂₂-Alkylmono-und - oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 6 bis 24, vorzugsweise 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare^{®} oder Plantaren® zur Verfügung stehen, enthalten eine glucosidisch gebundene C₈-C₁₆-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade^{®} PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin^{®} VL 75 im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Emulgatoren können beispielsweise Silikonemulgatoren enthalten sein. Diese können beispielsweise aus der Gruppe der Alkylmethicon-copolyole und/oder Alkyl-Dimethiconcopolyole gewählt werden, insbesondere aus der Gruppe der Verbindungen, welche gekennzeichnet sind durch die folgende chemische Struktur: 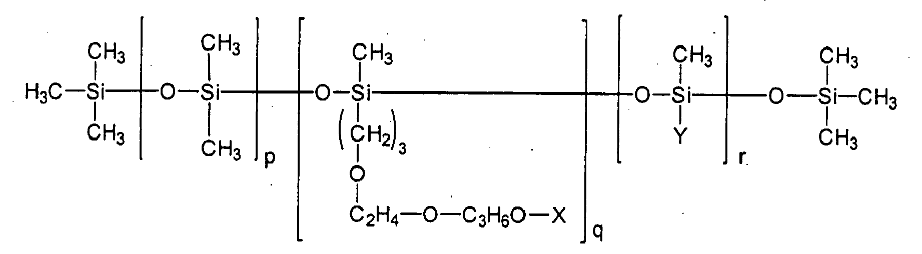 bei welcher X und Y unabhängig voneinander gewählt werden aus der Gruppe H (Wasserstoff) sowie der verzweigten und unverzweigten Alkylgruppen, Acylgruppen und Alkoxygruppen mit 1-24 Kohlenstoffatomen, p eine Zahl von 0-200 darstellt, q eine Zahl von 1-40 darstellt, und r eine Zahl von 1-100 darstellt.

Ein Beispiel für besonders vorteilhaft im Sinne der vorliegenden Erfindung zu verwendenden Silikonemulgatoren sind Dimethiconcopolyole, welche von Evonik Goldschmidt unter den Warenbezeichnungen AXIL® B 8842, ABIL® B 8843, ABIL® B 8847, ABIL® B 8851, ABIL® B 8852, ABIL® B 8863, ABIL® B 8873 und ABIL®B 88183 verkauft werden.

Ein weiteres Beispiel für besonders vorteilhaft im Sinne der vorliegenden Erfindung zu verwendende grenzflächenaktiven Substanzen ist das Cetyl PEG/PPG-10/1 Dimethicone (Cetyl Dimethiconcopolyol), welches von EvonikGoldschmidt unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopo- lyol, welches von Evonik Goldschmidt unter der Warenbezeichnung ABIL®EM 97 und ABIL®WE 09 verkauft wird.

Weiterhin hat sich als ganz besonders vorteilhaft der Emulgator Lauryl PEG/PPG-18/18 Methicone (Laurylmethiconcopolyol) herausgestellt, welcher unter der Warenbezeichnung Dow Corning® 5200 Formulation Aid von der Gesellschaft Dow Corning Ltd. erhältlich ist. Weiterhin vorteilhaft ist ein Silikonemulgator mit der INCI Bezeichnung Cyclopentasiloxane and PEG/PG-18-18 Dimethicone", der beispsielsweise unter dem Handelsnamen Dow Corning® 5225 C Formulation Aid erhältlich ist.

Ein weiterer vorteilhafter Silikonemulgator ist Octyl Dimethicon Ethoxy Glucosid der Firma Wacker. Für eine erfindungsgemäße Wasser-in-Silikonöl-Emulsion können alle bekannten für diesen Emulsionstyp verwendeten Emulgatoren eingesetzt werden. Erfindungsgemäß besonders bevorzugte Wasser-in-Silikon-Emulgatoren sind dabei Cetyl PEG/PPG- 10/1 Dimethicone und Lauryl PEG/PPG-18/18 Methicone [z.B. ABIL® EM 90 Evonik Goldschmidt), DC5200 Formulation Aid (Dow Corning)] sowie beliebige Mischungen aus beiden Emulgatoren.

Ein geeigneter anionischer O/W Emulgator ist z.B das unter der INCl Bezeichung erhältliche Produkt Disodium Cetearyl Sulfosuccinate (Handelsname Eumulgin® Prisma, Cognis GmbH).

### Tenside

In einer Ausführungsform der Erfindung enthalten die erfindungsgemäßen Zubereitungen als grenzflächen-aktive Verbindungen mindestens ein Tensid. Als grenzflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Typische Beispiele für nichtionische Tenside sind Fettalkoholpolyglycolether, Alkylphenolpolyglycolether, Fettsäurepolyglycolester, Fettsäureamidpolyglycolether, Fettamin-polyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als **zwitterionische Tenside** werden solche oberflächenaktiven Verbindungen bezeichnet, die im Molekül mindestens eine quartäre Ammoniumgruppe und mindestens eine -COO⁽⁻⁾-oder -SO₃⁽⁻⁾ -Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammonium-glycinate, beispielsweise das Kokosacylaminopropyldimethylammoniumglycinat, und 2-Alkyl-3-carboxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren (beispielsweise unter dem Handelsnamen Dehyton®DC kommerziell erhältlich), N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂-₁₈-Acylsarcosin. Weiterhin geeignten sind Derivate von N-Alkyliminodipropionsäuren, wie beispielsweise N-Lauryl-beta-Iminopropionate, kommerziell erhätlich unter dem Handelsnamen Deriphat® 160 C. Weiterhin geeignet sind Amphoacetate, wie z.B. Cocoamphoacetate (z.B. Dehyton® MC) oder Cocoamphodiacetate (wie z.B. Dehyton® DC).

Anionische Tenside sind gekennzeichnet durch eine wasserlöslich machende, anionische Gruppe wie z. B. eine Carboxylat-, Sulfat-, Sulfonat-, Citrat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze. Besonders geeignete anionische Tenside sind Glyceryl Stearate Citrate (wie z.B. kommerziell erhältlich unter den Handelsnamen Imwitor®370, Imwitor® 372P, Axol®C,62 or Dracorin®CE 614035) oder Glyceryl Stearat Lactat Verbindungen. Beispiel für ein geeignetes Alkylsulfat ist Sodium Cetearyl Sulfate (Handelsname Lanette® E), Beispiel für ein geeingetes Phosphate ist Potassium Cetyl Phosphate (Handelsname Amphisol® K). Beispiel für ein geeignetes Acylglutamat ist Sodium Stearoyl Glutamate (Handelsname z.B. Eumulgin® SG). Ein weiteres Beispiel für ein geeignetes anionische Tensid ist Sodium Lauryl Glucose Carboxylate (Handelsname Plantapon® LGC).

Als **kationische Tenside** sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Geeignete pseudo kationische Tenside sind beispielsweise Stearylaminopropyl Dimtheylamine (kommerziell erhältlich unter dem Handelsnamen Dehyquart® S18 oder Incromine® SB oderTegoAmide®S18). Weiterhin können die sehr gut biologisch abbaubaren quaternären Esterverbindungen, wie beispielsweise die unter dem Warenzeichen Stepantex^{®} vertriebenen Dialkylammoniummethosulfate und Methylhydroxyalkyldialkoyloxyalkylammoniummethosulfate und die entsprechenden Produkte der Dehyquart^{®}-Reihe, als kationische Tenside eingesetzt werden. Unter der Bezeichnung "Esterquats" werden im allgemeinen quaternierte Fettsäuretri-ethanolaminestersalze verstanden. Sie können den erfindungsgemäßen Zubereitungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar. Geeignete kationische Tenside sind beispielsweise Dipalmitoylethyl Hydroxyethylmonium Methosulfate (Handelsname Dehyquart®C4046), Distearoylethyl Hydroxyethylmonium Methosulfate (Handelsname Dehyquart®F75), Dicocoylethyl Hydroxyethylmonium Methosulfate (Handelsname Dehyquart® L80), Behentrimonium Chloride (Handelsname Varisoft® BT), Distearyldimonium Chloride (Handelsname Varisoft® TA 100), Palmitamidopropyltrimonium Chloride (Handelsname Varisoft® PATC).

### Wachskomponente b-2)

In einer Ausführungsform der Erfindung enthalten die erfindungsgemäßen Zubereitungen mindestens eine Wachskomponente. Die erfindungsgemäßen Zubereitungen enthalten den/die Wachskomponente(n) in einer Menge von 0 bis 40 Gew.-%, insbesondere von 0 bis 20 Gew.-%, vorzugsweise 0,1 bis 15 Gew.-% und insbesondere 0,1 bis 10 Gew.-% bezogen auf das Gesamtgewicht der Zubereitung.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß einsetzbar ist eine Wachskomponente oder ein Gemisch von Wachskomponenten, die bei 30 C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind so genannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter, Shea Butter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten TriglyceridGemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata AB und Novata B (Gemisch aus C12-C18-Mono-, Di- und Triglyceriden) sowie Cutina® HVG (Hydrogenated Vegetable Glycerides) oder Cutina® GMS (Glycerylstearat).

Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C14-C22-Fettalkohole, die beispielsweise von der Cognis Deutschland GmbH unter der Bezeichnung Lanette 16 (C16-Alkohol), Lanette 14 (C14-Alkohol), Lanette O (C16/C18-Alkohol) und Lanette 22 (C18/C22-Alkohol) vermarktet werden. Fettalkohole verleihen den Zubereitungen ein trockeneres Hautgefühl als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar.

### Polymere b-3)

In einer Ausführungsform der Erfindung enthalten die erfindungsgemäßen Zubereitungen mindestens ein Polymer. Die erfindungsgemäßen Zubereitungen enthalten das/die Polymere(n) in einer Menge von 0 bis 20 Gew.-%, vorzugsweise 0,05 bis 18 Gew.-% vorzugsweise 0,05 bis 15 Gew.-%, besonders bevorzugt 0,05 bis 10 Gew.-%, insbesondere 0,1 bis 1 Gew.-% bezogen auf das Gesamtgewicht der Zubereitungen. In einer bevorzugten Ausführungsform der Erfindung enthalten die erfindungsgemäßen Zubereitungen das Polymer/die Polymere in einer Menge von 0,1 bis 5 Gew.-%, insbesondere 0,1 bis 3 Gew.-%, insbesondere 0,1 bis 2 Gew.-% bezogen auf das Gesamtgewicht der Zubereitung.

Geeignete **kationische Polymere** sind beispielsweise kationische Cellulosederivate, wie z. B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium hydroxy-propyl hydrolyzed collagen (Lamequat^{®}L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine^{®}/Sandoz), Copolymere der Acrylsäure mit Dimethyldiallylammoniumchlorid (Merquat^{®} 550/Chemviron), Polyaminopolyamide, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z. B. Mirapol^{®} A-15, Mirapol^{®} AD-1, Mirapol^{®} AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Besonders geeignete anionische Polymer sind solche mit der INCI Bezeichnung Carbomer, wie z.B die Carbopol Typen 980, 980,981,1382,2984,5984 sowie die unter den Handelsnamen Rheocare®C plus and Rheocare®400 erhältlichen Produkte. Weiterhin geeignete anionische Polymere sind solche mit dem INCl Namen Acrylates/C10-30 Alkyl Acrylate Crosspolymer (Handelsnamen z.B. Pemulen®TR, Pemulen® TR 2, Carbopol®Ultrez), Acrylates Copolymer (Handelsnamen z.B. Rheocare TTA, TTN, TTN-2), Acrylamide/Sodium Acrylate Copolymer (Handelsnamen z.B. Cosmedia®ATC), Sodium Polyacrylate (Handelsnamen z.B. Cosmedia® ATH, Cosmedia®SP), Polyacrylamides (Handelsnamen z.B. Sepigel® 305 or Sepigel® 501). Bevorzugte anionische Polymere sind Polyacrylsäure Homo- und Copolymere.

Weiterhin geeignete Polymere sind Silicone Elastomer Gums, wie z.B. Silikone Elastomer Gemische, wie z.B. Gemische mit den INCI Bezeichnungen Cycolpentasiloxane (and) Dimethiconol (and) Dimethicone Crosspolymer (Handelsname Dow Corning®DC 9027), Gemische mit der INCI Bezeichnung Isodecyl neopentanoate (and) Dimethicone / bisisobutyl PPG-20 Crosspolymer (Handelsname Dow Corning®DC EL 8051 IN), Gemische mit der INCI Bezeichnung Dimethicone / Vinyl Dimethicone Crosspolymer (and) C12-14 Pareth-12) (Handelsname Dow Corning®DC 9509) sowie Gemische mit der INCI Bezeichnung Dimethicone / Vinyl Dimethicone Crosspolymer (and) Silica (Handelsname Dow Corning®DC 9701 Cosmetic Powder).

Als Polymere eigen sich ebenso Polysaccharide, insbesondere Xanthan-Gum, Guar-Gum, Agar-Agar, Alginate und Tylosen sowie Tara Gum, Carraghenan, Sclerotium Gum und natürliche Cellulose.

### Weitere Ölkörper b-4)

Körperpflegemittel, wie Cremes, Körperöle, Lotionen und Milchen, enthalten üblicherweise eine Reihe weiterer Ölkörper und Emollients, die dazu beitragen, die sensorischen Eigenschaften weiter zu optimieren. Die Ölkörper (erfindungsgemäße Verbindungen plus weitere Ölkörper) sind üblicherweise in einer Gesamtmenge von 0,1 - 80, insbesondere 0,5 bis 70, bevorzugt 1 bis 60, insbesondere 1 bis 50 Gew.-%, insbesondere 1 bis 40 Gew.-%, vorzugsweise 5 - 25 Gew.-% und insbesondere 5 - 15 Gew.-% enthalten. Die weiteren Ölkörper sind üblicherweise in einer Menge von 0,1 bis 40 Gew.-% enthalten

Als weitere Ölkörper kommen beispielsweise Guerbetalkohole auf Basis von Fettalkoholen mit 6 bis 18, vorzugsweise 8 bis 10 Kohlenstoffatomen, in Frage sowie Ester wie Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol), Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylylether (Cetiol® OE), Ringöffnungsprodukte von epoxidierten Fettsäureestern mit Polyolen und Kohlenwasserstoffen oder deren Gemischen. Weiterhin geeignet sind Ester von 2-Propylheptanol mit n-Octansäure, wie z.B. kommerziell erhältlich unter dem Handelsnamen Cetiol®SenSoft (Cognis GmbH). Weiterhin geeignet sind Kohlenwasserstoffe, wie zum Beispiel Undecan und Tridecan. Weiterhin geeignet sind Alkane, wie z.B. die Gemische mit der INCI Bezeichnung Conocnut/Palm/Palm Kernel Oil Alkanes (Handelsname Vegelight 1214 der Fa. Biosynthesis).

Überraschenderweise wurde gefunden, dass sich die erfindungsgemäßen Verbindungen insbesondere eignen um öllösliche kristalline UV-Lichtschutzfilter zu solubilisieren.

Ein Gegenstand der Erfindung betrifft Zubereitungen, enthaltend wenigstens eine Verbindung gemäß Anspruch 1 und mindestens einen UV-Lichtschutzfilter, vorzugsweise einen öllöslichen UV-Lichtschutzfilter.

Erfindungsgemäß sind als UV-Lichtschutzfilter bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
➢ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
➢ 3-(4'-Trimethylammonium) benzyliden- bornan-2-on-methylsulfat (Mexoryl SO)
➢ 3,3'-(1,4-Phenylendimethin)-bis (7,7- dimethyl-2-oxobicyclo-[2.2.1] heptan-1-methansulfonsäure) and salts (Mexoryl SX)
➢ 3-(4'-Sulfo)-benzyliden-bornan-2-on and salts (Mexoryl SL)
➢ Polymer von N-{(2und 4)- [2-oxoborn-3-yliden)methyl}benzyl]acrylamid (Mexoryl SW)
➢ 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl SL)
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoe-säureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4- diyl)diimino] bis(benzoesäure-2- ethylhexylester) (Uvasorb® HEB);
➢ 2,2(-Methylen-bis(6-(2H-benzotriazol-2-yl)-4-(1,1,3,3-tetramethyl-butyl)phenol) (Tinosorb M);
➢ 2,4-Bis[4-(2-ethylhexyloxy)-2- hydroxyphenyl]-6-(4- methoxyphenyl)-1,3,5- triazin (Tinosorb S);
➢ Propan-1,3-dione, wie z.B. 1-(4-tert.Butylphenyl)-3-(4`methoxyphenyl)propan-1,3-dion;
➢ Ketotricyclo(5.2.1.0)decan-Derivate, wie in der EP 0694521 B1 beschrieben;
➢ Dimethicodiethylbenzalmalonate (Parsol SLX).

Als wasserlösliche UV - Filter kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ 2,2(-(1,4-Phenylen)bis(1H-benzimidazol- 4,6-disulfon-säure, Mononatriumsalz) (Neo Heliopan AP)
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bornylidenmethyl)-benzolsulfonsäure und 2-Methyl-5-(2-oxo-3-bornyliden)sulfonsäure und deren Salze.

In einer bevorzugten Ausführungsform der Erfindung enthalten die Zubereitungen mindestens einen öllöslichen UV-Lichtschutzfilter sowie mindestens einen wasserlöslichen UV-Lichtschutzfilter.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Als UV-Lichtschutzfilter eignen sich insbesondere die gemäß Annex VII der Kommissions Direktive (in der **Fassung Commission Directive 2005/9/EC of 28 January 2005 amending Council Directive 76/768/EEC,** concerning cosmetic products, for the purposes of adapting Annexes VII thereof to technical progress) zugelassen Stoffe, auf die hier explizit Bezug genommen wird.

Die erfindungsgemäßen Zubereitungen können auch unlösliche Lichtschutzpigmente, nämlich feindisperse Metalloxide bzw. Salze enthalten. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 8/1996, S. 543-548 **sowie** Parf.Kosm. 80. Jahrgang, Nr. 3/1999, S. 10 bis 16 zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

In einer bevorzugten Ausführungsform der Erfindung enthalten die Zubereitungen mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate, 3-(4'-Trimethylammonium) benzyliden-bornan-2-on-methylsulfat, 3,3'-(1,4-Phenylendimethin)-bis(7,7-dimethyl-2-oxobicyclo-[2.2.1]heptan-1-methansulfonsäure) und ihre Salze, 3-(4'-Sulfo)-benzyliden-bornan-2-on und ihre Salze, Polymer von N-{(2und 4)- [2-oxoborn-3-yliden)methyl}benzyl]acrylamid, 2-(2H-Benzotriazol-2-yl)-4-methyl-6-(2-methyl-3-(1,3,3,3-tetramethyl-1-(trimethylsilyloxy)disiloxanyl)propyl)phenol, Dimethicodiethyl benzalmalonate und ihren Mischungen.

Diese UV-Lichtschutzfilter sind beispielsweise unter den folgenden Handelsnamen kommerziell erhältlich:
NeoHeliopan®MBC (INCl: 4-Methylbenzylidene Camphor; Hersteller: Symrise); NeoHeliopan® BB (INCl: Benzophenone-3, Hersteller: Symrise); Parsol®1789 (INCl: Butyl Methoxydibenzoylmethane, Hersteller: Hoffmann-La Roche (Givaudan); Tinosorb®S (INCl: Bis-Ethylhexyloxyphenol Methoxyphenyl Triazine); Tinosorb®M (INCl: Methylene Bis-Benzotriazolyl Tetramethylbutylphenol): Herstelller: Ciba Specialty Chemicals Corporation; Uvasorb®HEB (INCl: Diethylhexyl Butamido Triazone, Hersteller: 3V Inc.), Uvinul®T 150 (INCI: Ethylhexyl Triazone, Hersteller: BASF AG); Uvinul® A plus (INCl: Diethylamino Hydroxybenzoyl Hexyl Benzoate: Hersteller: BASF AG; Mexoryl® SO: 3-(4'-Trimethylammonium) benzyliden- bornan-2-on-methylsulfat, INCl: Camphor Benzalkonium Methosulfate; Mexoryl®SX: 3,3'-(1,4-Phenylendimethin)-bis (7,7- dimethyl-2-oxobicyclo-[2.2.1] heptan-1-methansulfonsäure), CTFA: INCl Terephthalylidene Dicamphor Sulfonic Acid; Mexory® SL: 3-(4'-Sulfo)-benzyliden-bornan-2-on, INCI Benzylidene Camphor Sulfonic Acid; Mexoryl®SW: Polymer von N-{(2und 4)-[2-oxoborn-3-yliden)methyl}benzyl]acrylamid, INCl Polyacrylamidomethyl Benzylidene Camphor; Mexoryl®SL: 2-(2H-Benzotriazol-2-yl)-4-methyl-6-(2-methyl-3-(1,3,3,3-tetramethyl-1-(trimethylsilyloxy) disiloxanyl)propyl) phenol; INCl: DROMETRIZOLE TRISILOXANE; Parsol® SLX: Dimethicodiethylbenzalmalonate, INCl Polysilicone-15.

Die erfindungsgemäßen Zubereitungen können die UV-Lichtschutzfilter in Mengen von 0,5 bis 30 Gew.-%, vorzugsweise 2,5 bis 20 Gew.-%, besonders bevorzugt 5 - 15 Gew.-% - bezogen auf die Zubereitung - enthalten.

### Weitere Inhaltsstoffe

Als **Verdickungsmittel** eignen sich beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox). Ein geeigneter Verdicker ist beispielsweise das unter den Handelsnamen Cosmedia® Gel CC erhältliche Produkt mit der INCI Bezeichnung Dicaprylyl Carbonate, Stearalkonium Hectorite and Propylene Carbonate. Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen. **Desodorierende Wirkstoffe / Antiperspirantien** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker. Als Insekten-Repellentien **kommen beispielsweise N,N-Diethyl-m-toluamid, 1,2-Pentandiol oder** 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage. Als **Selbstbräuner** eignet sich Dihydroxyaceton oder Erythrulose. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage. Als **Konservierungsmittel** eignen sich beispielsweise Phenoxyethanol, Formaldehydlösung, Parabene, Pentandiol, Chlorphenesin, Caprylylglykol, Ethylhexylglycerine oder Sorbinsäure sowie die unter der Bezeichnung Surfacine® bekannten Silberkomplexe und die in Anlage 6, Teil A und B der Kosmetikverordnung aufgeführten weiteren Stoffklassen. Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage. Als **Perlglanzwachse oder Perlglanz-Verbindungen,** insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Stearyl Citrate, Cyclodextrin, Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen. Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Ein geeignetes Überfettungsmittel ist beispielsweise die Mischung von Cocoglucosiden und Glyceryl Oleate (kommerziell erhältlich als Lamesoft® PO65 von Cognis GmbH).

Geeignete Füllstoffe sind Substanzen, die beispielsweise die sensorischen oder kosmetischen Eigenschaften einer Zubereitung verbessern und die beispielsweise ein samtiges oder seidiges Gefühl erzeugen oder verstärken (sog. Skin-Sensory Modifier). Geeignete Füllstoffe sind Stärke und Stärkederivate (wie z.B. Tapioka Stärke, Aluminium Starch Octenyl Succinate, Sodium Octenyl Succinat, Distärke Phosphat), Pigmente, die nicht hauptsächlich als UV-Filter oder Farbstoffe dienen (wie z.B. Bornitrid) und/oder Aerosil^{®} (CAS-Nr. 7631-86-9), und/oder Talkum, sowie beispielsweise Polymethyl Methacrylate (z.B. Cosmedia^{®} PMMA V8/V12), Silica (z. B. Cosmedia^{®} SILC), Stearalkonium Hectorite (wie im kommerziell erhältlichen Produkt Cosmedia® Gel CC enthalten) sowie HDI/Trimethylol Hexyllactone Crosspolymer (wie im kommerziell erhältlichen Produkt Cosmedia^{®} CUSHION enthalten).

Als **Stabilisatoren** können Metallsalze von Fettsäuren, wie z. B. Magnesium-, Aluminium-und/oder Zinkstearat bzw. -ricinoleat eingesetzt werden. Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Die erfindungsgemäßen Zubereitungen sowie die Verbindung gemäß Anspruch 1 eignen sich insbesondere in kosmetischen und/oder pharmazeutischen Zubereitungen zur Benetzung oder Imprägnierung oder Beschichtung von Gebrauchs- und Hygienetüchern, die zur Körperreinigung und/oder zur Körperpflege eingesetzt werden.

Als Gebrauchs- und Hygienetücher seien exemplarisch genannt: Tissues, Papiere, Wipes, Vlies-Produkte, Schwämme, Puffs, Pflaster und Bandagen, die ihren Einsatz im Bereich der Hygiene und Pflege finden. Dies können sein Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte sowie Selbstbräunungs-Wipes.

### Verfahren zur Herstellung

Ein weiterer Gegenstand der vorliegenden Erfindung betrifft ein Verfahren zur Herstellung von Verbindungen der allgemeinen Formel (I), bei dem man Alkohole der allgemeinen Formel R₁-OH und/oder R₂-OH in Gegenwart von Katalysatoren kondensiert, wobei R₁ und R₂ die oben angegebene Bedeutung besitzen.

Als Katalysatoren haben sich insbesondere saure Katalysatoren wie KHSO₄, Schwefelsäure oder Alkylschwefelsäurehalbester und Sulfonsäuren oder deren Ester als geeignet erwiesen. Es können auch Heteropolywolframsäuren als Katalysatoren eingesetzt werden. Die sauren Katalysatoren reagieren im Verlauf der Veretherung mit den Alkoholen teilweise oder vollständig unter Esterbildung ab.

Besonders bevorzugt sind Sulfonsäuren mit der folgenden, R⁵SO₃H(IV) in der R⁵ für einen Alkylrest mit 1 bis 4 Kohlenstoffatomen oder einen gegebenenfalls alkylsubstituierten Arylrest steht.

Typische Beispiele für geeignete Sulfonsäuren sind Methansulfonsäure, Ethansulfonsäure, Propansulfonsäure, Butansulfonsäure, Sulfoölsäure oder p-Toluolsulfonsäure, Trifluormethansulfonsäure, insbesondere Methansulfonsäure, Alkylbenzolsulfonsäure und Naphthalinsulfonsäure. Als weitere geeignete Sulfonsäuren haben sich darüber hinaus Anlagerungsprodukte von Natriumhydrogensulfit an olefinische Systeme, insbesondere an Maleinsäure, erwiesen. Ein typisches Beispiel hierfür ist die Sulfobernsteinsäure.

Die Dehydratisierung der Alkohole R₁-OH und/oder R₂-OH kann in Gegenwart von 0,01 bis 10 Gew.-%, vorzugsweise 0,01 bis 3 Gew.-%, insbesondere 0,01 bis 1 Gew.-% - bezogen auf die Alkohole - der Sulfonsäuren erfolgen.

Zur Durchführung des erfindungsgemäßen Verfahrens werden die Alkohole R₁-OH und/oder R₂-OH vermischt und auf Reaktionstemperatur gebracht. Die Reaktion wird bevorzugt bei einer Temperatur von 120 bis 260°C, besonders bevorzugt 140 °C bis 250 °C, insbesondere 180 bis 220° C, durchgeführt.

Die Reaktion kann unter Inertgasatmosphäre, z.B. unter Stickstoffatmosphäre, durchgeführt werden. Die Reaktion kann bei Normaldruck oder erhöhtem Druck bis etwa 4 bar durchgeführt werden. Erhöhter Druck ist insbesondere dann erwünscht, wenn Alkohole eingesetzt werden, deren Siedetemperatur unter der Reaktionstemperatur liegt.

Ein weiterer Gegenstand der vorliegenden Erfindung betrifft ein Verfahren zur Herstellung von Verbindungen der allgemeinen Formel (I), bei dem man ein Alkohol der allgemeinen Formel R₁-OH und/oder R₂-OH in Gegenwart eines Alkylhalogenids der allgemeinen Formel R₁-X und/oder R₂-Y umsetzt, wobei R₁ und R₂ die oben angegebene Bedeutung besitzen und X und Y ausgewählt sind aus der Gruppe bestehend aus Chlorid, Fluorid, Bromid und lodid.

Das erfindungsgemäße Verfahren wird üblicherweise bei 20 bis 250 °C, insbesondere bei 25 bis 150 °C, insbesondere bei 40 bis 90 °C, vorzugsweise bei 60 bis 90 °C durchgeführt. Das Verfahren kann bei Normaldruck (1bar) sowie bei erhöhtem Druck, d.h. bei 2 bis 10 bar, vorzugsweise bei 3 bis 5 bar durchgeführt werden.

Das Verfahren wird üblicherweise in Gegenwart alkalischer Verbindungen, beispielsweise Alkalimetallhydroxide, wie Natriumhydroxid oder Kalimhydroxid erhalten.

### Beispiele

### (1) Herstellbeispiele

### Herstellbeispiel 1: Propylheptyl-methylether

949,8g Propylheptanol (6mol) und 320,0g NaOH (50%ig) (4mol) wurden am Wasserabscheider für 10 Stunden auf 220 °C erhitzt. Zu dem entstandenen, viskosen Alkoholat wurden 596,4g Methyliodid (4mol) gegeben und weitere 10 h auf 80°C erhitzt. Das Produkt wurde zunächst durch Filtration von gebildetem Natriumiodid befreit und anschließend mit einer Füllkörperkolonne destilliert. Das Produkt fällt als farbloses Öl mit geringer Viskosität an.

### Herstellbeispiel 2: Di(2-Propylheptyl)ether

600 g Propylheptanol (3,8 mol), 12 g Trifluormethansulfonsäure (50 %ig, 0,04 mol) und 12g Unterphosphorige Säure (0,09 mol) wurden am Dephlegmator langsam auf 160 °C erhitzt und diese Temperatur wurde für 6h gehalten. Nach dem Abkühlen wurde mit 25 g Natronlauge (50 %ig, 313 mmol) neutralisiert und anschließend wurde das Produkt mit einer Füllkörperkolonne destilliert. Das Produkt siedet bei 120 °C bei 0,001 bar und fällt als farbloses Öl an.

### (2) Hautverträglichkeit

Der gemäß Herstellbeispiel 2 erhaltene Di(2-Propylheptyl)ether wurde in einem okklusiven epicutanen 24h Patch Test gegen den kommerziell erhältlichen Di-n-octylether verglichen. Dazu wurden 22 Probanden mit jeweils 75 µl der zu testenden Substanzen auf dem Rücken aufgetragen (Patch Test System: Finn Chambers® mit okklusiven Filterbehältern auf Scanpor®), als Blindkontrolle diente Wasser, als Positivkontrollen eine 0,5%ige Lösung von SDS (Sodium Dodecyl Sulfate). 24 nach Auftrag wurde der Patch entfernt, nach weiteren 6 Stunden erfolgte die erste visuelle Bewertung, weitere Bewertungen erfolgten nach 24, 48 und 72 Stunden nachdem der Patch entfernt wurde. grag

Die Bewertung erfolgte auf einer Skala von 0 (keine Reaktion) bis 4 (starke Reaktion) in den Kategorien: Erythem Bildung, Ödem Bildung, Schuppen-Bildung (= Squamation) und Rissbildung (= Fissuration). Aus den so erhaltenen Werten wurde ein sog. Total Irriation Score RSS Wert gebildet.

| Testsubstanz | RSS |
|---|---|
| Blindwert | 0,14 |
| Positivkontrolle | 7,95 |
| Di-n-alkylether (Cetiol® OE) | 7,1 |
| Di(2-Propylheptyl)ether gemäß Herstellbeispiel 2 | 3,62 |

Wie aus der Tabelle ersichtlich, zeigt die erfindungsgemäße Verbindung gegenüber dem Stand der Technik eine deutlich verbesserte Hautverträglichkeit.

### (3) Kosmetische Zubereitungen

Die nachfolgenden kosmetischen Zubereitungen wurden sowohl mit dem erfindungsgemäßen Ether gemäß Herstellbeispiel 1 (Propylheptyl-methylether) als auch mit dem erfindungsgemäßen Ether gemäß Herstellbeispiel 2 (Di(2-Propylheptyl)ether) hergestellt. Alle Angaben in Gew.-% bezogen auf das Gesamtgewicht der Zubereitung.

**Tabelle 1: O/W Body Care Emulsionen**

| **Inhaltsstoffe C-Creme, L - Lotion** | **1** | **2** | **3** | **4** | **5** | **6** | **7** | **8** | **9** | **10** | **11** |
|---|---|---|---|---|---|---|---|---|---|---|---|
| | **C** | **C** | **C** | **L** | **C** | **L** | **L** | **C** | **L** | **C** | **C** |
| Eumulgin^{®} VL 75 | | | | | | | | 2,0 | | 1.5 | |
| Dehymuls^{®} PGPH | | 0.6 | | | | | | | | | |
| Generol^{®} R | | | 0.5 | | | | | | | | |
| Eumulgin^{®} B2 | | | 2,0 | | | | | | 2,0 | | |
| Tween^{®} 60 | | | | 0.2 | | | | | | | |
| Cutina^{®} E 24 | | | | 0.2 | | | | | | | |
| Hostaphat^{®} KL 340 N | | | | | | | | | 0.5 | | |
| Lanette^{®} E | | | | | | | | 0.6 | | | |
| Amphisol^{®} K | | | 0.2 | | | | | | | | |
| Sodium Stearate | | | | | 0.5 | | | | | | |
| Emulgade^{®} PL 68/50 | 3,0 | | | | | | 2,0 | | | | 1.2 |
| Eumulgin^{®} SG | 0,2 | | | | 0,2 | 0,3 | | | | | |
| Eumulgin^{®} Prisma | | 0,2 | | | | | 0,2 | | | 0,2 | 0,5 |
| Inwitor 372 P | | | | | | 3,0 | | | | 3,0 | |
| Tego^{®} Care CG | 0.7 | | | | | | | | | | |
| Tego^{®} Care 450 | | | | | 3 | | 1,0 | | | 1,0 | |
| Cutina^{®} PES | 2.5 | 2 | 3 | | | 2 | | 1.7 | 2.5 | | 1.2 |
| Cutina^{®} MD | | 1 | | 3 | 5 | | 2 | | | 3 | |
| Lanette^{®} 14 | | | | 1 | | | | 4 | | | 4 |
| Lanette^{®} O | 4.5 | | 4 | | 1 | | | | | | 2 |
| Novata^{®} AB | | 1 | | | | | | | | | 1 |
| Emery^{®} 1780 | | | | | 0.5 | 0.5 | | | | | |
| Lanolin, water-free, USP | | | | | | | 1.1 | | | | |
| Cosmedia^{®} DC | | 1.5 | 2 | | | 1.5 | 2 | | 1.5 | 1.5 | |
| Cetiol^{®} SB 45 | | | 1.5 | | | | 2 | | | | |
| Cegesoft^{®} C 17 | | | | | | | | | | | 2 |
| Myritol^{®} PC | | | | | 5 | | | | | | |
| Myritol^{®} 331 | 2 | 5 | 1 | | | 6 | | 6 | | | |
| Finsolv^{®} TN | | | 2 | | | 2 | | | | | |

| **Propylheptyl-methylether oder Di(2-Propylheptyl)-ether** | 4 | 3 | 4 | 5 | 4 | 4 | 4 | 6 | 8 | 3 | 5 |
|---|---|---|---|---|---|---|---|---|---|---|---|
| Cetiol^{®} Sensoft | 2,0 | | | | | 2,0 | | | | 3,0 | |
| Cetiol^{®} CC | | 3 | | | | | 4 | | | | 5 |
| Cetiol^{®} OE | | | 2,0 | | | | | | 4 | | |
| Dow Corning DC^{®} 245 | | | 2 | | 1 | 1 | | | | | |
| Dow Corning DC^{®} 2502 | | | | | 2 | 1 | | | | | |
| Prisorine^{®} 3758 | | | | | | 1 | | | | | |
| Silicone Oil Wacker AK^{®} 350 | 0.5 | 0.5 | 0.5 | | | 1 | | | | | |
| Cetiol^{®} 868 | | | | | 2 | | 4 | | | | |
| Cetiol^{®} J 600 | 2 | | 3 | | 3 | 2 | | | | 5 | |
| Ceraphyl^{®} 45 | | | | | | | 3 | | | | |
| Mineral Oil | | | | 9 | | | | | | | |
| Cetiol^{®} SN | | | 5 | | | | | | | | |
| Cetiol^{®} B | | | | | | | | 4 | | 2 | |
| Eutanol^{®} G | | 2 | | 3 | | | | | | | |
| Cetiol^{®} PGL | | | | | | | | | 5 | 5 | |
| Dry Flo ^{®} Plus | 5 | | | | | | 1 | | | | |
| SFE 839 | 5 | | | | | | | | | | 2 |
| Almond Oil | | | | | | | 1 | | | | |
| Insect Repellent^{®} 3535 | | 2 | 4 | | | 2 | | | | 3 | |
| N,N-Diethyl-m-toluamide | | 2 | | | | | | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Tocopherol / Tocopheryl Acetate | 1 | | | | | | | | | | |
| Veegum^{®} Ultra | | | | | | | | | 1 | | |
| Keltrol^{®} T | | | 0.4 | | | | | | 0.5 | | |
| Cosmedia^{®} SP | | 0.3 | | 0.2 | 0,2 | | | | 0.2 | 0.3 | |
| Pemulen^{®} TR 2 | 0.3 | | | | | | | 0.3 | | | |
| Carbopol^{®} Ultrez 10 | | | | | | 0.2 | | | | | |
| Rheocare® C Plus | | | 0.3 | | 0.2 | | | | | | |
| Ultragel™ 300 | | | | | | | | | 0,2 | | |
| Ethanol | | | | | | | | | | 10 | |
| Butylene glycol | | | | 4 | 3 | | 2 | 5 | 2 | | |
| Glycerin | 2 | 5 | 5 | | 3 | 3 | 2 | | 4 | | 3 |
| Water, Preservatives, NaOH | ad 100, q.s., pH 6,5 - 7,5 | | | | | | | | | | |

**Tabelle 2: O/W Body Care Emulsionen**

| **Inhaltsstoffe C-Creme, L - Lotion** | **12** | **13** | **14** | **15** | **16** | **17** | **18** | **19** | **20** | **21** | **22** |
|---|---|---|---|---|---|---|---|---|---|---|---|
| | **C** | **C** | **L** | **C** | **L** | **C** | **C** | **L** | **L** | **L** | **C** |
| Eumulgin^{®} VL 75 | | | 1 | | | | | | | | 1 |
| Generol^{®} R | | | | | | 0.3 | | | | | |
| Eumulgin^{®} B2 | | | | | | | | | | 2 | |
| Tween^{®} 60 | | | | 2 | | | | | | 1 | |
| Cutina^{®} E 24 | | | | 0.5 | | | | | | 1 | |
| Lanette^{®} E | 0.5 | | | | | | | | | | |
| Amphisol^{®} K | | 0.5 | | | | | | | 0,1 | | |
| Sodium Stearate | | | | | 1 | | | | | | |
| Emulgade^{®} PL 68/50 | | 3 | | | | 3,0 | | 1 | 2 | | |
| Eumulgin^{®} SG | | | | | | | 0,5 | | | | 0,5 |
| Eumulgin^{®} Prisma | | | 0,5 | | | 0,2 | | 0,2 | | | |
| Inwitor 372 P | 3 | 2 | 3 | | 3 | | 1 | 1 | | | |
| Tego^{®} Care 450 | | | | | 1 | 2,0 | 3.8 | 1 | 1 | | |
| Cutina^{®} PES | 2 | | 1 | | 2.5 | 2 | | 1.2 | | 1.5 | 3 |
| Cutina^{®} MD | 3 | 1 | | 4 | | | | | | | |
| Lanette^{®} 14 | | 2 | | | 1 | | | 2 | | 1 | |
| Lanette^{®} O | 2 | | | 2 | | 3 | 1 | | 1 | 1 | 6 |
| Novata^{®} AB | | | | | | | | | 1 | 1 | |
| Emery^{®} 1780 | | | | | | | | | | | 0.5 |
| Lanolin, water-free, USP | | | | | | 4 | | | | | |
| Cosmedia^{®} DC | | | 1 | | | 1.5 | | | 1 | 1 | |
| Cetiol^{®} SB 45 | | | | | | | 2 | | | | |
| Cegesoft^{®} C 17 | 4 | | | | | | | | | | |
| Myritol^{®} PC | 6 | | | | | 5 | | | 5 | | |
| Myritol^{®} 331 | | | 5 | | | | 7 | | | 10 | 3 |
| Finsolv^{®} TN | | 5 | | 4 | 5 | | | | | | 1 |

| **Propylheptyl-methylether oder Di(2-Propylheptyl)-ether** | 5 | 2 | 4 | 6 | 2 | 5 | 4 | 3 | 3 | 8 | 2 |
|---|---|---|---|---|---|---|---|---|---|---|---|
| Cetiol^{®} Sensoft | | 2 | | 3 | | | | | | | |
| Cetiol^{®} CC | | | 4 | | | | | 3 | | | |
| Cetiol^{®} OE | 2.5 | | | | | | 2 | | 5 | | |
| Dow Corning DC^{®} 245 | | | | | 1 | 3 | | | | | 2 |
| Dow Corning DC^{®} 2502 | | 1 | | | 1 | | | | | | 3 |
| Prisorine^{®} 3758 | 3 | | | | | | | | | | 2 |
| Silicone Oil Wacker AK^{®} 350 | | | | | 1 | | | | | | 1 |
| Cetiol^{®} 868 | | 2 | | | | | | | | | |
| Cetiol^{®} J 600 | | 2 | | 2 | | | | | | | |
| Ceraphyl^{®} 45 | | | | | | | 3 | | | | |
| Cetiol^{®} SN | | | | 5 | | | | | | | |
| Cetiol^{®} B | | | | | | 5 | | 4 | | | 3 |
| Eutanol^{®} G | | 3 | | | 5 | | | | | | |
| Cetiol^{®} PGL | | | | | | | | 5 | 2 | | |
| Dry Flo^{®} Plus | | 1 | | | | | | | | | 1 |
| SFE 839 | 1 | 1 | | | | | | | | | |
| Almond Oil | | | | | | 2 | | | | | |
| Photonyl^{®} LS | | | | | | 2 | | | | | |
| Panthenol | | | | | | 1 | | | | | |
| Bisabolol | 0.2 | | | | | | | | | | |
| Tocopherol / Tocopheryl Acetate | 1 | | | | | | | | | | |
| Veegum^{®} Ultra | | | | | | | | | 1 | | |
| Keltrol^{®} T | | | | | | | | | 0.5 | | |
| Cosmedia^{®} SP | 0.1 | | 1 | | 0.2 | 0.2 | 0.2 | 0.2 | | | 0.5 |
| Carbopol^{®} ETD 2001 | | | | 0.3 | | | | | | | |
| Pemulen^{®} TR 2 | | | | | | 0.3 | | | | | |
| Rheocare® C Plus | 0,2 | 0.3 | | | | | | | | | |
| Ultragel^{™} 300 | | | | 0,4 | | 0,3 | | | | 0,4 | |
| Ethanol | | 5 | | 8 | | | | | | | 10 |
| Butylene glycol | 5 | | | 3 | 3 | | | | | 8 | |
| Glycerin | 2 | 4 | 3 | 3 | | 7 | 5 | 3 | 5 | | |
| Water, Preservatives, NaOH | ad 100, q.s., (pH 6,5 - 7,5) | | | | | | | | | | |

**Tabelle 3: O/W Body Care Emulsionen**

| **Inhaltsstoffe C-Creme, L-Lotion, SC=Sprühbare Creme** | **23** | **24** | **25** | **26** | **27** |
|---|---|---|---|---|---|
| | SC | C | C | L | C |
| Dehyquart^{®} C 4046 | 6 | | | 3 | |
| Cutina^{®} GMS-SE | | | | | 5.5 |
| Cutina^{®} FS 45 | | | | | 1.5 |
| Eumulgin^{®} B2 | | 1 | | | |
| Eumulgin^{®} SG | | | 0,2 | | |
| Eumulgin^{®} Prisma | | 0,2 | | | |
| Inwitor 372 P | | | 2 | | |
| Cutina^{®} PES | 3 | 2 | 2 | 2 | 2 |
| Cutina^{®} MD | | 1,5 | | | |
| Cosmedia^{®} DC | | | | 0.5 | |
| Cegesoft^{®} PS 6 | | | | 4.5 | |
| Cegesoft^{®} SH | | 7 | 3 | | |
| Myritol^{®} 331 | | | 5 | 4.5 | |

| **Propylheptyl-methylether oder Di(2-Propylheptyl)-ether** | 4 | 5 | 4 | 3 | 4 |
|---|---|---|---|---|---|
| Cetiol^{®} Sensoft | | 2 | | | |
| Cetiol^{®} CC | | | 3 | | |
| Cetiol^{®} OE | | 1 | | | |
| Silicone Oil Wacker AK^{®} 350 | | | | | 0.5 |
| Paraffin Liquid | | | | | 2 |
| Isopropyl Palmitate | | | | 2 | |
| Cetiol^{®} 868 | | 2 | 4 | | |
| Cetiol^{®} SN | 4 | | | | 3 |
| Eutanol^{®} G | | | | | 3 |
| Almond Oil | | 7 | | | |
| Panthenol | 1 | 0.2 | 1 | | |
| Bisabolol | 1 | | | | |
| Tocopherol / Tocopheryl Acetate | 0.2 | | | | |
| Keltrol^{®} T | 1 | | | | |
| Ultragel^{™} 300 | 0,1 | | | 0.45 | |
| Cosmedia^{®} SP | | 1 | 0.7 | | |
| Glycerin | 2 | 5 | 5 | 5 | |
| Water, Preservatives, NaOH | ad 100, | q.s. | | | |

**Tabelle 4: W/O Body Care Emulsionen**

| **Inhaltsstoffe (INCI)** | **1** | **2** | **3** | **4** | **5** | **6** | **7** | **8** | **9** | **10** | **11** |
|---|---|---|---|---|---|---|---|---|---|---|---|
| **L = Lotion, C = Creme** | C | L | C | L | C | L | L | L | C | C | C |
| Dehymuls^{®} PGPH | 1 | 2 | 1 | 2 | 3 | 1 | 1 | 2 | | | 1 |
| Monomuls^{®} 90-018 | 2 | | | | | | | | 2 | | 2 |
| Lameform^{®} TGI | 4 | 1 | | | 3 | | | 1 | 4 | 3 | |
| Abil^{®} EM 90 | | | | | | | 4 | | 1 | | |
| Isolan GPS | | | 2 | | 2 | | | | | 1 | |
| Isolan^{®} PDI | | | | | | 4 | | | | | 1 |
| Glucate^{®} DO | | | | 3 | | | | | | | |
| Arlacel^{®} 83 | | | 4 | | | | | | | | |
| Dehymuls^{®} LE | | 1 | 1 | 2 | | | | | | 1 | 1 |
| Dehymuls^{®} HRE 7 | | | | | | | | 4 | | 1 | |
| Zinc Stearate | 2 | 1 | | 1 | 1 | | | 1 | 1 | 1 | |
| Microcristalline Wax | | | 5 | 5 2 | 5 2 | 2 | | | | | 5 |
| Bees wax | 4 | | | 1 | | | | 1 | 4 | 7 | |
| Tego Care^{®} CG | | | | | 1 | | | | | | 0,5 |
| Prisorine^{®} 3505 | | | 1 | 1 | | 1 | 1 | | | | 1 |
| SFE^{®} 839 | | | | | | | 3 | | | | |
| Emery^{®} 1780 | 1 | | | | | | | | | | 1 |
| Anhydrous Lanolin USP | | | 5 | | | | | | | 4 | |

| **Propylheptyl-methylether oder Di(2-Propylheptyl)-ether** | 3 | 4 | 2 | 6 | 6 | 2 | 2 | 6 | 3 | 8 | 1 |
|---|---|---|---|---|---|---|---|---|---|---|---|
| Cegesoft^{®} C 17 | | | 3 | | | | | | | 1 | |
| Myritol^{®} PC | | | | | | 2 | 2 4 | 4 | | | |
| Myritol^{®} 331 | 6 | | | | 2 | 6 | 2 | | | | 8 |
| Finsolv^{®} TN | | | | 5 | | 2 | 5 | | | | |
| Cetiol^{®} A | | 6 | | | | 4 | | | | | |
| Cetiol^{®} Sensoft | | | | 6 | 4 | | | | | 4 | |
| Cetiol^{®} CC | | 8 | | | 2 | 2 | 2 | | | | 5 |
| Cetiol^{®} SN | | 5 | | | | | | 3 | | | |
| Cetiol^{®} OE | 3 | | | | 4 | | 2 | | 4 | 2 | |
| Dow Corning DC^{®} 244 | | | | | 1 | | 2 | | | | |
| Dow Corning DC^{®} 2502 | | | 1 | | 2 | | | | | | |
| Prisorine^{®} 3758 | | | | | 3 | | | | | | |
| Silicon Oil Wacker AK^{®} 350 | | | | 4 | | | | 3 | | | |
| Cetiol^{®} 868 | | | | | | | | | | 2 | 7 |
| Cetiol^{®} J 600 | | | 4 | | | 2 | | | | | |
| Ceraphyl^{®} 45 | | | | 2 | | | | 2 | | 6 | |
| Mineral oil | | | | | 4 | | | | | | |
| Cetiol^{®} B | | | 2 | 4 | | | | | | 3 | |
| Eutanol^{®} G 16 | | 1 | | | | | | | | 3 | |
| Eutanol^{®} G | | | 3 | | | | | 8 | | | |
| Cetiol^{®} PGL | | | | | | 4 | | | 9 | | |
| Almond Oil | | | | | 1 | | 5 | | | | |
| Insect Repellent^{®} 3535 | 2 | | | | | | | | | | |
| Unirep^{®} U-18 | | | | 3 | | | | 5 | | | |
| Photonyl^{®} LS | 2 | 2 | | | | | | | | | |
| Panthenol | 1,0 | | | | | | | | | | |
| Bisabolol | 0,2 | | | | | | | | | | |
| Copherol^{®} 1250 C | 1 | | | | | | | | | | |
| MgSO₄ x 7 H₂O | 1 | | | | | | | | | | |
| Bentone^{®} 38 | | | | | 1 | | | | | | |
| Propylene Carbonate | | | | | 0,5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Water, Preservative | ad 100, q.s. | | | | | | | | | | |

**Tabelle 5: O/W Sun Care Emulsionen**

| **Inhaltsstoffe** | **1** | **2** | **3** | **4** | **5** | **6** | **7** | **8** | **9** | **10** | **11** |
|---|---|---|---|---|---|---|---|---|---|---|---|
| **C - Creme, L - Lotion** | **L** | **C** | **S** | **L** | **C** | **L** | **L** | **C** | **L** | **C** | **L** |
| Eumulgin^{®} VL 75 | 2,0 | | | | | | | 2 | | | 2 |
| Eumulgin^{®} B2 | | | | 0.5 | | | | | | | |
| Tween^{®} 60 | | | | 0.2 | | | | | | | |
| Myrj^{®} 51 | | | | 0.5 | | | | | | | |
| Cutina^{®} E 24 | | | | 0.1 | | | | | | | |
| Hostaphat^{®} KL 340 N | | | | | | | | | 1.6 | | |
| Lanette^{®} E | | | 0.3 | | | | | | | | |
| Amphisol^{®} K | | | | | | | | | | 1 | |
| Sodium Stearate | | | | | | | 1 | | | | |
| Emulgade^{®} PL 68/50 | | 2 | 1 | | | 2 | 2 | | | 2 | |
| Imwitor 372 P | | 2 | | | | 1 | | 2 | | | |
| Eumulgin^{®} SG | | 0,5 | | | | 0,1 | | 0,2 | | | |
| Eumulgin^{®} Prisma | 0,1 | | | | 0,75 | | | | | | |
| Tego^{®} Care 450 | | | | | | 2 | | | | 1 | 2.5 |
| Cutina^{®} PES | 2 | | 2.5 | 1 | 2.5 | | 2.5 | | 2.5 | 1.7 | 1.5 |
| Cutina^{®} MD | 2 | | 1 | 2 | | | 2 | | | 6 | |
| Lanette^{®} 14 | 1 | | | 1 | | | | 2 | | | 2 |
| Lanette^{®}O | 1 | 6 | | | 5 | 2 | | 2 | | | |
| Cosmedia^{®} DC | 1 | 1.5 | | 1 | 1 | | 2 | 2 | | | 2 |
| Antaron^{®}V 216 | | | 2 | | | 1.5 | | | 1 | 1 | |
| Emery 1780 | | | | | 0.5 | 0.5 | | | | | |
| Lanolin, water-free USP | | | | | | | 5 | | | | |
| Myritol^{®} PC | | | | | 5 | | | | | | |
| Myritol^{®} 331 | | | 8 | | | 6 | | 10 | | 2 | |
| Finsolv^{®} TN | | | 1 | | | | | 1 | | | |

| **Propylheptyl-methylether oder Di(2-Propylheptyl)-ether** | 5 | 2 | 3 | 5 | 3 | 4 | 3 | 2 | 5 | 2 | 5 |
|---|---|---|---|---|---|---|---|---|---|---|---|
| Cetiol^{®} Sensoft | | 2,5 | | | 2 | | | | 3 | | |
| Cetiol^{®} CC | | | 2 | | | | 1 | | | | |
| Cetiol^{®} OE | | | 3 | | | | | | 2 | 3 | |
| Dow Corning DC^{®} 244 | 4 | | 1 | | | | | 2 | | | 2 |
| Dow Corning DC^{®} 2502 | | 1 | | | 2 | | | | | | |
| Squatol^{®} S | | | | | | | 4 | | | | |
| Silicone Oil Wacker AK^{®} 350 | | 2 | | | | | | | | | |
| Cetiol^{®} 868 | | | | | 2 | | 4 | | | | 2 |
| Cetiol^{®} J 600 | | | | | 3 | 2 | | | | 5 | |
| Mineral Oil | | | | 4 | | | | | | | |
| Cetiol^{®} B | | | 1 | | | | | | | 2 | |
| Eutanol^{®} G | | | | 2 | | | | | 4 | | |
| Eutanol^{®} G 16 | 4 | | | | | 4 | | | | | |
| Cetiol^{®} PGL | | 5 | | | | | | | | 5 | |
| Almond Oil | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol / Tocopheryl Acetate | 1 | | | | | | | | | | |
| Photonyl^{®} LS | | | | | | | | | | | |
| Neo Heliopan^{®} AP (Na-salt) | | 1 | | | | | | | 1 | | |
| Neo Heliopan^{®} Hydro (Na-salt) | 2 | | 2.2 | | | | | | 1 | | |
| Neo Heliopan^{®} 303 | 3 | 5 | 9 | 4 | | | | | | | |
| Neo Heliopan^{®} BB | | | | | 1 | | | | | | 2 |
| Neo Heliopan^{®} MBC | 2 | | | 3 | | 2 | 2 | 2 | | | 1 |
| Neo Heliopan^{®} OS | | | | | | | | | 10 | 7 | |
| Neo Heliopan^{®} E 1000 | | 7.5 | | 6 | | | | | | | 6 |
| Neo Heliopan^{®} AV | | | 7.5 | | | 7.5 | 4 | 5 | | | |
| Uvinul^{®} A Plus | | | | 2 | 1 | | | | | | |
| Uvinul^{®} T 150 | 2 | | | | 2.5 | | | 1 | | | |
| Tinosorb^{®} M | | | 3 | | | | 2 | | | | 3 |
| Tinosorb^{®} S | | | 1 | | | | 1,5 | | | | |
| Uvasorb^{®} HEB | | 1 | | | 1 | | | | | | |
| Parsol^{®} 1789 | | 1 | 1 | | | | 2 | | 2 | 2 | |
| Zinkoxid NDM | 10 | | 5 | | | 10 | | 3 | | 5 | 4 |
| Eusolex^{®} T 2000 | | | | | 5 | | 3 | 3 | | | 4 |
| Veegum^{®} Ultra | 1.5 | | 0.75 | | | | | 1 | 1 | | |
| Keltrol^{®} T | 0.5 | | 0.25 | | | | | 0.5 | 0.5 | | |
| Cosmedia^{®} SP | 0,1 | 0.5 | | | 0.5 | | 0.2 | 0.2 | | 0.2 | 0.2 |
| Ultragel^{™} 300 | | | | 0.2 | | 0.2 | | | 0.1 | | |
| Rheocare^{®} C plus | | | | | | | | | | 0.3 | 0.2 |
| Ethanol | | | | | | | | | | 10 | |
| Butylene glycol | | 2 | | 4 | 3 | | 2 | 5 | 2 | | 2 |
| Glycerin | 5 | 5 | 5 | | 3 | 3 | 2 | | 4 | | 3 |
| Preservatives, NaOH, Water | q.s.ad 100 | | | | | | | | | | |

**Tabelle 6: O/W Sun Care Emulsionen**

| **Inhattssioffe** | **12** | **13** | **14** | **15** | **16** | **17** | **18** | **19** | **20** | **21** | **22** |
|---|---|---|---|---|---|---|---|---|---|---|---|
| **C-Creme, L-Lotion** | **L** | **C** | **L** | **C** | **L** | **C** | **S** | **C** | **C** | **L** | **L** |
| Eumulgin^{®} VL 75 | | | 4 | | 1.8 | | | | | | |
| Eumulgin^{®} B2 | | | | | | | | | | 0.2 | |
| Tween^{®} 60 | | | | | | | | | | 0.3 | |
| Cutina^{®} E 24 | | | | | | | | | | 0.5 | |
| Hostaphat^{®} KL 340 N | | | | | | | | | | | 0.5 |
| Imwitor 372 P | 2 | | | 2 | | | 2 | | 2.0 | | |
| Eumulgin^{®} SG | | | | 0,1 | | 0,2 | | | | | |
| Eumulgin^{®} Prisma | | 0,3 | 0,2 | | | | | | | | |
| Lanette^{®} E | | | | | | | 0.1 | | 0.5 | | |
| Amphisol^{®} K | 0.5 | | | | | | | 1 | | | |
| Sodium Stearate | | | | | 1 | | | | | | |
| Emulgade^{®} PL 68/50 | | 1.5 | | 2 | | 3 | | 2 | | | |
| Tego^{®} Care 450 | 1 | | | | | 2 | | 2 | 0.8 | | |
| Cutina^{®} PES | 2 | 2 | 2.5 | 1.5 | 2 | 2 | 2.5 | 3 | | 1.5 | 1.5 |
| Cutina^{®} MD | 1 | | | 4 | 1 | 3 | | | 5 | | 1 |
| Lanette^{®} 14 | | 2 | | | | | | | | 1 | |
| Lanette^{®} O | | 2 | | 2 | | | | 2 | 1 | 1 | |
| Allianz^{®} OPT | 1 | | | 1 | 1 | | | 2 | | | 2 |
| Cosmedia^{®} DC | | 1.5 | 2 | | | 1.5 | 2 | | 1.5 | 1.5 | |
| Emery^{®} 1780 | | | | 1 | 1 | | | | | | |
| Lanolin, water-free, USP | | | | | | 1 | 1 | | | | |
| Myritol^{®} PC | | | | | | | | | 3 | | |
| Myritol^{®} 331 | 12 | | 12 | | | 8 | 8 | | | 5 | 3 |
| Finsolv^{®} TN | | | | | 3 | | | | 3 | | |

| **Propylheptyl-methylether oder Di(2-Propylheptyl)-ether** | 4 | 2 | 3 | 5 | 3 | 2 | 4 | 3 | 2 | 5 | 3 |
|---|---|---|---|---|---|---|---|---|---|---|---|
| Cetiol^{®} Sensoft | | | 3 | | | 5 | | | | | 2 |
| Cetiol^{®} CC | 2 | | | | | | 1 | | | | |
| Cetiol^{®} OE | | | | | 2 | | | | | | 2 |
| Dow Corning DC^{®} 244 | | | | | 1 | | | | | | |
| Dow Corning DC^{®} 2502 | | 1 | | | | | | 3 | | | |
| Ceraphyl^{®} 45 | | | | | | | | | | 2 | 2 |
| Silicone oil Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Mineral Oil | | | | 5 | | | | | | | |
| Cetiol^{®} B | 4 | | 4 | | | | | 4 | | | |
| Eutanol^{®} G | | 3 | | | | 3 | | | | | |
| Eutanol^{®} G 16 S | 10 | | | | | | | | | | |
| Cetiol^{®} PGL | | | | | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopheryl Acetate | 1 | | | | | | | | | | |
| Neo Heliopan^{®} Hydro (Na-salt) | | | | | | | | | | 3 | |
| Eusolex^{®} OCR | 6 | | 9 | | 5 | 7 | 9 | | 4 | | 7 |
| Neo Heliopan^{®} AP (Na-salt) | | | | 0.5 | | 1 | | | | | |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} A PLUS | | | | | 1 | | 2 | | | | |
| Uvinul^{®} T 150 | 1 | | | | | | | | 1.3 | 1 | 1 |
| Tinosorb^{®} M | | | 6,5 | | | | | | | 4 | |
| Tinosorb^{®} S | | | 1 | | 2 | | | | | | |
| Uvasorb^{®} HEB | 1 | | | | | | | | | | 2 |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Cosmedia^{®} SP | | | 0.2 | | | | 0.1 | | | | |
| Pemulen^{®} TR 2 | | 0.3 | | 0.3 | | | | 0.2 | | | |
| Ultragel^{™} 300 | | | | | | | | | | 0.2 | 0.3 |
| Rheocare® C Plus | | | | 0.3 | | | 0,1 | | | | |
| Ethanol | | 5 | | 8 | | | | | | | |
| Butylene glycol | 1 | | | 3 | 3 | | | | | 8 | 1 |
| Glycerin | 2 | 4 | 3 | 3 | | 3 | 3 | 3 | 5 | | 3 |
| Water / Preservatives / NaOH | ad 100/ q.s./ q.s | | | | | | | | | | |

**Tabelle 7: W/O Sun Care Emulsionen**

| **Inhaltsstoffe** | **23** | **24** | **25** | **26** | **27** | **28** | **29** | **30** | | **31 32** | **34** |
|---|---|---|---|---|---|---|---|---|---|---|---|
| **C-Creme, L-Lotion** | **C** | **L** | **C** | **L** | **C** | **L** | **L** | **L** | **L** | **C** | **C** |
| Dehymuls^{®} PGPH | 4 | 3 | 1 | 3 | 2 | 1 | 1 | 1 | | | |
| Monomuls^{®} 90-018 | | 1 | 2 | | | | | | 2 | 4 | |
| Lameform^{®} TGI | 2 | | | | 3 | | | | | 1 | 3 |
| Abil^{®} EM 90 | 2 | | | | | | 4 | | 1 | | 1 |
| Isolan GPS | | | 4 | | 3 | | | 2 | | | |
| Isolan^{®} PDI | | | | | | 4 | | 2 | | | |
| Zinc Stearate | 1 | | | 1 | 1 | | | 1 | | 1 | |
| Beeswax | 1 | | 5 | 1 | 3 | | | 2 | | 7 | 5 |
| Tego^{®} Care CG | | | | | 1 | | | | | | 0.5 |
| Cutina^{®} PES | | | 2 | | | 1 | 1 | | | | |
| Prisorine^{®} 3505 | | | 1 | | | 1 | 1 | | | | 1 |
| Cosmedia^{®} DC | 3 | 4 | 2 | 1 | 1 | 2 | 2 | 2 | 3 | 1 | 1 |
| Myritol^{®} 331 | 2 | | | | 3 | 3 | | | | | 8 |
| Finsolv^{®} TN | | | | 2 | | | | | | | |

| **Propylheptyl-methylether oder Di(2-Propylheptyl)-ether** | 5 | 4 | 2 | 3 | 4 | 3 | 5 | 5 | 4 | 4 | 5 |
|---|---|---|---|---|---|---|---|---|---|---|---|
| Cetiol^{®} Sensoft | | | | 3 | | | 5 | | | 3 | |
| Cetiol^{®} CC | 5 | | | | | 2 | | | 2 | 3 | |
| Tegosoft^{®} DEC | | 4 | | | 2 | | | | | | |
| Cetiol^{®} OE | | | | | 4 | | 5 | | | 2 | |
| Dow Corning^{®} DC 244 | | | 3 | | | | 2 | | 4 | | |
| Dow Corning^{®} DC 2502 | 1 | | 1 | | 2 | 1 | | | | | 1 |
| Silicone oil Wacker AK 350 | | 1 | | 4 | | | | 3 | | | |
| Cetiol^{®} PGL | | 3 | | | | 4 | | | 4 | | |
| Copherol^{®} F 1300 | | | | | 1 | | | | | | |
| MgSO₄ * 7H₂O | | | | | 1 | | | | | | |
| Neo Heliopan^{®} Hydro (Na-salt) | 2 | | 2.2 | | 3 | 3 | | | 1 | 1 2 | 2 |
| Neo Heliopan^{®} 303 | | 5 | | | | | | | 4 | 4 | 4 |
| Uvasorb^{®} HEB | 1 | | | 1 | 1 | | | | | | 2 |
| Neo Heliopan^{®} MBC | 2 | | | | | 2 | 2 | 2 | | | |
| Uvinul^{®} A Plus | | | | | 2 | | | | 3 | 3 | |
| Neo Heliopan^{®} AP (Na-salt) | | 2 | 2 | | 1 | | | | 1 | | 6 |
| Neo Heliopan^{®} AV | 3 | | 4 | 6 | 4 | 7.5 | 4 | 5 | | | 1 |
| Uvinul^{®} T 150 | 1 | 1 | | | 2.5 | | | 1 | | | |
| Parsol^{®} 1789 | 2 | 1 | | | | | 2 | | 2 | 2 | |
| Zinkoxid NDM | | | | | | 10 | | 3 | | | 4 |
| Tinosorb^{®} M | | 3 | | 3 | | | | 2 | | 2 | |
| Tinosorb^{®} S | | 3 | | 3 | | | | 2 | | 2 | |
| Eusolex^{®} T Aqua | | | 8 | | | | | 5 | | | |
| Eusolex^{®} T 2000 | | | | | 5 | | 3 | 3 | | | 4 |
| Ethanol | | | | | | | | | | 8 | |
| Glycerin | 5 | 3 | 3 | 3 | 5 | 3 | 2 | 3 | 10 | 4 | 3 |
| Water, Preservatives | ad 100, q.s. | | | | | | | | | | |

**Tabelle 8: W/O Sun Care Emulsionen**

| **-** | **12** | **13** | **14** | **15** | **16** | **17** |
|---|---|---|---|---|---|---|
| Dehymuls^{®} PGPH | 1 | 1 | | | 1 | 1 |
| Dehymuls^{®} LE | 1 | 2 | 1 | 1 | 1 | 1 |
| Abil^{®} EM 90 | | | | 4 | | |
| Isolan GPS | 3 | | 1 | 1 | | |
| Isolan^{®} PDI | | | 4 | | 2 | |
| Zinc Stearate | | 1 | | | 1 | |
| Beeswax | | 1 | | | 5 | |
| Cutina^{®} PES | | 1 | | 1 | | |
| Prisorine^{®} 3505 | | | 1 | 1 | | |
| Cosmedia^{®} DC | 4 | 1 | 2 | 2 | 2 | 3 |
| Myritol^{®} 331 | | | 3 | | | |
| Finsolv^{®} TN | | 2 | | | | |

| **Propylheptyl-methylether oder Di(2-Propylheptyl)-ether** | 4 | 3 | 3 | 5 | 5 | 4 |
|---|---|---|---|---|---|---|
| Cetiol^{®} CC | | | 2 | 2 | 2 | 2 |
| Cetiol^{®} Sensoft | | 2 | | 2 | | 4 |
| Tegosoft^{®} DEC | 4 | 3 | | 5 | | |
| Cetiol^{®} OE | 2 | | | 5 | | |
| Dow Corning^{®} DC 244 | | | | 2 | | 4 |
| Dow Corning^{®} DC 2502 | | | 1 | | | |
| Silicone oil Wacker AK 350 | 1 | 4 | | | 3 | |
| Cetiol^{®} PGL | 3 | 3 4 | 4 | | | 4 |
| Copherol^{®} F 1300 | | | | 1 | | |
| MgSO₄ * 7H₂O | | | | 1 | | |
| Neo Heliopan^{®} Hydro (Na-salt) | | | 3 | | | 1 |
| Neo Heliopan^{®} 303 | 5 | | | | | 4 |
| Uvasorb^{®} HEB | | 1 | | | | |
| Neo Heliopan^{®} MBC | | | 2 | 2 | 2 | |
| Uvinul^{®} A Plus | | | | | | 3 |
| Neo Heliopan^{®} AP (Na-salt) | 2 | | | | | 1 |
| Neo Heliopan^{®} AV | | 6 | 7.5 | 4 | 5 | |
| Uvinul^{®} T 150 | 1 | | | | 1 | |
| Parsol^{®} 1789 | 1 | | | 2 | | 2 |
| Zinkoxid NDM | | | 10 | | 3 | |
| Tinosorb^{®} M | 3 | 3 | | | 2 | |
| Tinosorb^{®} S | 3 | 3 | | | 2 | |
| Eusolex^{®} T Aqua | | | | | 5 | |
| Eusolex^{®} T 2000 | | | | 3 | 3 | |
| Glycerin | 3 | 3 | 3 | 2 | 3 | 10 |
| Water, Preservatives | ad 100, q.s. | | | | | |

**Tabelle 9: Dekorative Kosmetics - O/W Foundations**

| **Inhaltsstoffe** | **1** | **2** | **3** | **4** | **5** | **6** | **7** | **8** |
|---|---|---|---|---|---|---|---|---|
| Cutina^{®} GMS-SE | 5,5 | | | | | | | 3,0 |
| Emulgade^{®} PL 68/50 | | 5,0 | | | | 2,0 | | |
| Eumulgin^{®} VL 75 | | | | 3,0 | | | 5,0 | |
| Tego Care^{®} 450 | | | | | | 2,0 | 2,0 | |
| Codesta^{®} F-50 | | | | | 6,0 | | | |
| Amphisol^{®} K | | | | 2,0 | | | | |
| Lanette^{®} E | | 0,25 | | | | | | |
| Eumulgin^{®} SG | | | | | 1,0 | | 1 | |
| Eumulgin^{®} Prisma | | | | | | 1,0 | | 0,75 |
| Imwitor 372 P | | 2 | | | | | 1 | |
| Cutina^{®} FS 45 | 1,5 | | | | | | | |
| Eumulgin^{®} B 2 | | | 2,0 | | | | | |
| Cutina^{®} PES | 2,0 | 1,0 | 2,0 | 4,0 | 2,0 | 1,0 | 2,5 | 2,0 |
| Lanette^{®} O | | | 2,0 | | | | | 1,0 |
| Cutina^{®} MD | | 0,5 | 3,0 | 3,0 | | | | |
| Cetiol^{®} LC | 4,0 | | | | | | | |
| Cosmedia^{®} DC | 0,5 | | | 1,0 | | | | 1,0 |
| **Propylheptyl-methylether oder Di(2-Propylheptyl)-ether** | 4,0 | 5,0 | 4,0 | 2,0 | 7,0 | 5,0 | 10,0 | 4,0 |
| Cetiol^{®} Sensoft | 2,0 | | | | 3,0 | | | 2,0 |
| Tegosoft^{®} DEC | | 5,0 | | 2,0 | | 2,0 | | 2,0 |
| Cetiol^{®} CC | | | 2,0 | | 2,0 | | | |
| Dow Corning^{®} 245 | | 2,0 | | 2,0 | | | | |
| Eutanol^{®} G 16 | 4,0 | | | | | 3,0 | | |
| Myritol^{®} 331 | | 5,0 | | | 2,0 | 2,0 | 5,0 | |
| Uvinul^{®} T 150 | | | | 0,5 | | | | 0,5 |
| Uvasorb^{®} HEB | 2,0 | | | | | | 1,0 | 1,0 |
| Tinosorb^{®} M | | | 2,0 | | | | | 2,0 |
| Tinosorb^{®} S | | | | 3,0 | | | | 2,0 |
| Neo Heliopan^{®} AV | | | | 2,0 | | | 2,0 | |
| Heo Heliopan^{®} AP | | | | 1,0 | | | 1,0 | |
| Uvinul^{®} A Plus | | | 1 | | | | 2,0 | 2,0 |
| Microna^{®} Matte White | 5,0 | 5,0 | | 5,0 | 5,0 | 5,0 | | 5,0 |
| Microna^{®} Matte Black | 0,3 | 0,3 | 0,1 | 0,3 | 0,3 | 0,3 | 0,4 | 0,3 |
| Microna^{®} Matte Yellow | 3,0 | 3,0 | | 3,0 | 3,0 | 3,0 | 2,0 | 3,0 |
| Microna^{®} Matte Red | 0,6 | 0,6 | 1,0 | 0,6 | 0,6 | 0,6 | 1,0 | 0,6 |
| Ronasphere^{®} | 1,0 | 1,0 | | 1,0 | 1,0 | 1,0 | | 1,0 |
| Pigment White 6 | | | 6,0 | | | | 6,0 | |
| Dry Flow PC | | | | | | | 2,0 | 2,0 |
| Glycerin | 5,0 | 5,0 | 3,0 | 5,0 | 5,0 | 5,0 | 3,0 | |
| Cosmedia^{®} SP | | | 0,3 | | 0,2 | | | |
| Water, de-ionized, Preservative | ad 100 | | | | | | | |

**Tabelle 10: Dekorative Kosmetik - W/O Foundations**

| **Inhaltsstoffe** | **1** | **2** | **3** | **4** | **5** | **6** | **7** | **8** |
|---|---|---|---|---|---|---|---|---|
| Dehymuls^{®} PGPH | 5,5 | | 4,0 | | | | | 3,0 |
| Lameform^{®} TGI | | 5,0 | | | | 2,0 | | |
| Abil^{®} EM 90 | | | | 3,0 | | | 5,0 | |
| Isolan^{®} Gl 34 | | | | | | 2,0 | 2,0 | |
| Isolan^{®} PDI | | | | 1,0 | 6,0 | | | |
| Isolan^{®} GPS | 1,0 | 2,0 | | 1,0 | | | | |
| Admul^{®} WOL 1403 | | | | 2,0 | | | | |
| Dehymuls^{®} HRE 7 | | 1,0 | | | 1,0 | 1,0 | | |
| Monomuls^{®} 90-018 | 1,5 | | | | | | | 2,0 |
| Cutina^{®} PES | 2,0 | 1,0 | 2,0 | 4,0 | 2,0 | 1,0 | 2,5 | 2,0 |
| Cera Bellina | | | 2,0 | | | | | 2,0 |
| Beeswax | | | 2,0 | | | 2,0 | | 1,0 |
| Microcrystalline Wax | | 1,5 | 3,0 | 3,0 | | | | |
| Cetiol^{®} LC | 4,0 | 5,0 | | | | | | |
| Cosmedia^{®} DC | 1,0 | | | | 0,5 | | 1,0 | |
| **Propylheptyl-methylether oder Di(2-Propylheptyl)-ether** | 4,0 | 2,0 | 2,0 | 4,0 | 5,0 | 5,0 | 5,0 | 4,0 |
| Cetiol^{®} Sensoft | | 2,0 | | | 2,0 | | 5,0 | |
| Tegosoft^{®} DEC | | 3,0 | | | | 2,0 | | |
| Cetiol^{®} CC | | | | | 2,0 | | | 2,0 |
| Dow Corning^{®} 245 | | 2,0 | | 2,0 | | | | 2,0 |
| Eutanol^{®} G 16 | 4,0 | | | | 3,0 | 3,0 | | |
| Myritol^{®} 331 | | 5,0 | | | 2,0 | 2,0 | 5,0 | |
| Uvinul^{®} T 150 | | | | 0,5 | | | | 0,5 |
| Uvasorb^{®} HEB | | | 2,0 | | | | 1,0 | 1,0 |
| Tinosorb^{®} M | | | 2,0 | | | | | 2,0 |
| Tinosorb S | | | | 3,0 | | | | 2,0 |
| Neo Heliopan^{®} AV | | | | 2,0 | | | 2,0 | |
| Heo Heliopan^{®} AP | | | | 1,0 | | | 1,0 | |
| Uvinul^{®} A plus | | | 1,0 | | | | 2,0 | 2,0 |
| Microna^{®} Matte White | 5,0 | 5,0 | | 5,0 | 5,0 | 5,0 | | 5,0 |
| Microna^{®} Matte Black | 0,3 | 0,3 | 0,1 | 0,3 | 0,3 | 0,3 | 0,4 | 0,3 |
| Microna^{®} Matte Yellow | 3,0 | 3,0 | 3,5 | 3,0 | 3,0 | 3,0 | 2,0 | 3,0 |
| Microna^{®} Matte Red | 0,6 | 0,6 | 1,0 | 0,6 | 0,6 | 0,6 | 1,0 | 0,6 |
| Ronasphere^{®} | 1,0 | 1,0 | | 1,0 | 1,0 | 1,0 | | 1,0 |
| Pigment White 6 | | | 6,0 | | | | 6,0 | |
| Dry Flow PC | | | | | | | 2,0 | 2,0 |
| Glycerin | 5,0 | 5,0 | 3,0 | 5,0 | 5,0 | 5,0 | 3,0 | |
| Water, de-ionized, Preservative | ad 100 | | | | | | | |

**Tabelle 11: Dekorative Cosmetic - Lippenstifte**

| **Inhaltsstoffe** | **1** | **2** | | **3 4** |
|---|---|---|---|---|
| Cutina^{®} LM conc | | | 10,0 | 36,0 |
| Candelilla Wax | 9,39 | 5,0 | 10,0 | |
| Carnauba wax | 2,85 | 7,0 | 5,0 | |
| Beeswax | 1,86 | 5,0 | 4,0 | |
| Cutina^{®} PES | 3,2 | 5,0 | 6,4 | 4,5 |
| Cetiol^{®} MM | | | 5,0 | |
| Cosmedia^{®} DC | 5,0 | 4,0 | 2,0 | 6,0 |

| **Propylheptyl-methylether oder Di(2-Propylheptyl)-ether** | 7,0 | 6,0 | 3,0 | 5,0 |
|---|---|---|---|---|
| Cetiol^{®} Sensoft | 2,0 | | 4,5 | |
| Tegosoft^{®} DEC | 3,0 | 3,0 | 3,0 | 5,0 |
| Eutanol^{®} G | 10,97 | 12,0 | 12,0 | |
| Fitoderm^{®} | | | 4,0 | |
| Monomuls^{®} 90L 12 | | 3,0 | | |
| Dehymuls^{®} PGPH | | 4,0 | | |
| Castor Oil | 11,0 | 15,5 | 14,5 | 30,0 |
| Copherol^{®} F 1300 | 1,0 | 1,0 | 1,0 | 1,0 |
| Cosmetic white C47056 | 5,0 | 2,0 | 5,0 | |
| FDC Yellow 6 Al Lake C705270 | 7,0 | 7,0 | 8,0 | |
| DC Red 7 Ca Lake C 19003 | 6,0 | 4,5 | 1,1 | 2,9 |
| Irodin 100 Silverpearl | | | | 9,6 |
| Hydagen^{®} CMF | | 10,0 | | |
| Irwinol^{®} LS 9319 | 1,0 | | 3,0 | |
| Mineral Oil | 12,8 | | | |
| Petrolatum | 6,84 | 3,0 | | |
| Ceresin | 2,75 | | | |
| Microcrystalline Wax | 2,45 | | | |
| Colophane Claire type Y | 1,89 | | | |

**Tabelle 12: AP/Deo Konzepte**

| **Inhaltsstoffe (INCl)** | **1** | **2** | **3** | **4** | **5** | **6** | **7** |
|---|---|---|---|---|---|---|---|
| Glyceryl Stearate, Ceteareth-20, Ceteareth-12, Cetearyl Alcohol, Cetyl Palmitate | 6 | | | 4,5 | | 6 | |
| (Emulgade^{®} SE) | | | | | | | |
| Ceteareth-20 (Eumulgin^{®}B2) | | | | 1 | | | |
| Glyceryl Stearate Citrate (Imwitor 372 P) | | 4,0 | | | | | |
| Polyglyceryl-3 Diisostearate (Lameform^{®} TGI) | | | 3 | | | | |
| Cocoglycerides (Novata^{®} AB) | | | | | | | 4 |
| Stearyl alcohol (Lanette^{®} 18) | | | | | 10 | | |
| Hydrogenated Castor Oil (Cutina^{®} HR) | | | | | 3,7 | | 6,5 |
| Polyglyceryl-2 Dipolyhydroxystearate (Dehymuls^{®} PGPH) | | | 1 | | | | |
| Sodium Stearoyl Glutamate (Eumulgin^{®} SG) | | 0,2 | | | | | |
| Disodium Cetearyl Sulfosuccinate (Eumulgin^{®} Prisma) | 0,3 | | | | | | |
| Sodium Cetearyl Sulfate (Lanette^{®} E) | | | | | | 0,3 | |
| Pentaerythrityl Distearate (Cutina^{®} PES) | 5 | 1 | 2 | 1 | 4,7 | 5 | 4 |
| Behenyl Alcohol (Lanette^{®} 22) | 2 | 1 | | | | 4 | |

| **Propylheptyl-methylether oder Di(2-Propylheptyl)-ether** | 4 | 4 | 5 | 3 | 4 | 3 | 5 |
|---|---|---|---|---|---|---|---|
| Propylheptyl Caprylate (Cetiol^{®} Sensoft) | | 2 | | | 20 | | 10 |
| Dicaprylyl Carbonate (Cetiol^{®} CC) | | | 2 | | | | |
| Dicaprylyl Ether (Cetiol^{®} OE) | 2 | | | 2 | 5 | 3 | 4 |
| Cocoglycerides (Myritol^{®} 331) | | | | | | | |
| Diethylhexylcyclohexane (Cetiol^{®} S) | | | | 5 | 14,7 | | 25 |
| Cyclopentasiloxane | 3 | | 5 | | 14 | 3 | 14 |
| Cyclopentasiloxane and Dimethicone / Vinyldimethicone Crosspolymer SFE 839 (GE Bayer) | | | 3 | | | | |
| Dimethicone AK 350 | 1 | 2 | | | | | |
| Hydrogenated Dimer Dilinoleyl / Dimethylcarbonate Copolymer (Cosmedia^{®} DC) | 0,5 | | 1 | 1,5 | 1 | 2 | 1 |
| Triethyl Citrate (Hydagen^{®} C.A.T) | | | | 2 | | | |
| Tocopheryl Acetate | | | | | 1 | | |
| Aluminium Zirconium Tetrachlorohydrex GLY (Rezal 36) | 30 | | 40 | | 22,9 | 30 | 25 |
| Aluminum Chlorhydrate (Locron L) | | 20 | | 10 | | | |
| Chitosan (Hydagen^{®} DCMF) | 0,05 | | | | | | |
| Glycolic Acid | 0,02 | | | | | | |
| Glycerin | | | 5 | 5 | | | |
| Propylene Carbonate (Fluka) | | | | | | | 0,5 |
| Quaternium-18 Hectorite (Bentone 18) | | | | | | | 1 |
| Polyquaternium- 37 (Ultragel 37) | | 5 | | | | | |
| Talcum (Merck) | | | | | | 5 | 5 |
| MgSO₄x 7H₂O | | | 1 | | | | |
| Wasser, Perfume, Preservatives | q.s. | q.s. | q.s. | q.s. | q.s. | q.s. | q.s. |

1/2 - Antiperspirant / deo Creme, 3 - Antiperspirant Creme (W/0), 4 - Antiperspirant / Deo Spray, 5 - Antiperspirant Stift mit Vitamin E, 6 - Antiperspirant Creme, 7 - Antiperspirant Creme 'Soft Solid'

**Tabelle 13: Haarpflege Conditioner**

| **Inhaltsstoff (INCI)** | **1** | **2** | **3** | **5** | **6** | **7** | **8** |
|---|---|---|---|---|---|---|---|
| Structure^{®} XL (*) (Hydroxypropyl Starch Phosphate) | 5.0 | 5.0 | 5.0 | 4.0 | | | |
| Emulgade^{®} Sucro (Sucrose Polystearate, Hydrogenated Polyisobutene) | | | | | 1.0 | 1.0 | 1.0 |
| Dehyquart^{®} L 80 (Dicocoylethyl Hydroxyethylmonium Methosulfate, Propylene Glycol) | 2.6 | 1.3 | 2.0 | | | 0.5 | 0.5 |
| Dehyquart^{®} F 75 (Distearoylethyl Hydroxyethylmonium Methosulfate, Cetearyl Alcohol) | | | 2.0 | | | | |
| Dehyquart^{®} C 304 (Aqua, Cocamidopropyl-trimonium Methosulfate, Propylene Glycol) | | | | 3.7 | | 4.0 | 4.0 |
| **Propylheptyl-methylether oder Di(2-Propylheptyl)-ether** | 1.0 | 3.0 | 1.0 | 0.5 | 2.0 | 1.5 | 1.5 |
| Dehyquart^{®} A CA (Cetrimonium Chloride) | | | | | 4.0 | | |
| DC 200 (***) (Dimethicone) | | | | 0.5 | | | |
| Lanette^{®} O (Cetearyl Alcohol) | | | 1.0 | | 4.0 | | |
| Lamesoft^{®} TM Benz (Glycol Distearate, Coco Glucoside, Glyceryl Oleate, Glyceryl Stearate) | 4.0 | | | 1.0 | | | |
| Gluadin^{®} WLM (Hydrolyzed Wheat Protein) | 1.0 | 1.0 | | 1.0 | | | 0.3 |
| Glycerin | | | 0.5 | | | | |
| Gluadin^{®} Soy (Hydrolyzed Wheat Protein) | | 0.5 | | | | | |
| Cacao Butter (**) Theobroma Cacao (Cocoa) Seed Butter | | | 0.5 | | | | |
| Herbalia^{®} Balm Mint (Melissa Officinalis, Maltodextrin, Silica) | | 0.01 | | | | | 0.02 |
| Ultragel^{™} 300 (Polyquarternium-37) | | | | | | 0,2 | 0,2 |
| Perfume , preservative | q.s. | q.s. | q.s. | q.s. | q.s. | q.s. | q.s. |
| Wasser | Ad 100 | | | | | | |

| | | | | | | | |
|---|---|---|---|---|---|---|---|
| (*)National Starch, (**) Nederland, (***) Dow Corning; pH adjusted to 3.5 - 5.0 | | | | | | | |

**Tabelle 14: Haarpflege Conditioners**

| **Inhaltsstoff (INCI)** | **9** | **10** | **11** | **12** |
|---|---|---|---|---|
| Dehyquart^{®} L 80 (Dicocoylethyl Hydroxyethylmonium Methosulfate, Propylene Glycol) | 1.3 | 1.3 | | 1.0 |
| Dehyquart^{®} F 75 (Distearoylethyl Hydroxyethylmonium Methosulfate, Cetearyl Alcohol) | 1.3 | 1.3 | 1.3 | 1.5 |
| Lanette^{®} O (Cetearyl Alcohol) | 5.0 | 5.0 | 4.0 | 4.5 |

| **Propylheptyl-methylether oder Di(2-Propylheptyl)-ether** | 1.0 | 1.0 | 1.0 | 0.5 |
|---|---|---|---|---|
| Cetiol^{®} SB 45 Butyrospermum Parkii (Shea Butter) | 4.0 | 4.0 | 2.0 | 4.5 |
| Gluadin^{®} Almound (Hydrolyzed Sweet Almound Protein) | | | 0.1 | 0.5 |
| ASCO BTAC (Behentrimonium Chloride) | | | 1.3 | |
| DC 949 (****) Amodimethicone, Trideceth-12, Cetrimonium Chloride | | 1.0 | | |
| Cegesoft^{®} PFO (Passiflora Incarnata Seed Oil) | | | 2.0 | |
| Aloveria^{®} (Aloe Barbadensis) | 0.1 | | | |
| Sphingoceryl^{®} Veg: Octyldodecanol, Hydrogenated Coco Glycerides, Helianthus Annuus (Sunflower) Seed Extract | 1.0 | | | |
| Copherol^{®} 1250 (Tocopheryl Acetate) | 0.2 | | | |
| Ultragel^{™} 300 (Polyquarternium-37) | | 0,1 | | 0,2 |
| Perfume , preservative | q.s. | q.s. | q.s. | q.s. |
| Wasser | Ad 100 | | | |

| | | | | |
|---|---|---|---|---|
| (****) Dow Corning; pH adjusted to 3.5 - 5.0 | | | | |

**Tabelle 15: Haarpflege Conditioner**

| | | | | |
|---|---|---|---|---|
| | **13** | **14** | **15** | **16** |

| **Propylheptyl-methylether oder Di(2-Propylheptyl)-ether** | 10 | 10.6 | 43.6 | 30 |
|---|---|---|---|---|
| Myritol^{®} 318 (Caprylic Capric Triglyceride) | | | 43.6 | 20 |
| Cetiol^{®} ISL (Isostearyl Lactate) | | | | 40 |
| DC 1501 (*) (Cyclomethicone, Dimethiconol) | 69.5 | | | |
| Emery^{®} 3004 (Hydrogenated Polydecene) | | 67.8 | | |
| DC 345 (*) Cyclomethicone | 20 | | | |
| Versagel MC 750 (**) Isohexadecene, Ethylene/Propylene/Styrene Copolymer, Butylene/Ethylene/Styrene Copolymer | | 21.3 | | |
| DC 556 (*) Phenyl Trimethicone | 0.5 | | | |
| Wacker HDK H 20 (***): Phenyl Trimethicone | | | 12.5 | 10 |
| Ultragel^{™} 300 (Polyquarternium-37) | 0,2 | | | 0,2 |
| Perfume | q.s. | q.s. | q.s. | q.s. |

| | | | | |
|---|---|---|---|---|
| (*) Dow Corning, (**) Penreco, (***) Wacker | | | | |

**Tabelle 16: Haarpflege Conditioner**

| **Inhaltsstoffe (INCI)** | **17** | **18** | **19** | **20** | **21** |
|---|---|---|---|---|---|
| Cetearyl Alcohol (Lanette^{®} O) | 5,0 | 4,5 | | | |
| Glyceryl Stearate (Cutina^{®} MD) | 4,0 | | | 14,5 | |
| Cetearyl Alcohol (Lanette^{®} O) | | | | 7,0 | |
| Hydrogenated Castor Oil (Cutina^{®} HR) | | | | 2,5 | |
| Cetyl Palmitate (Cutina^{®} CP) | | 0,3 | | 7,0 | |
| Paraffin Oil | | | | 23,5 | |
| Vaseline | | | | 32,5 | |
| Wacker Silicon Oil AK 350 | | | | 0,5 | |

| **Propylheptyl-methylether oder Di(2-Propylheptyl)-ether** | 3,0 | 0,2 | 1,5 | 2,0 | 5,0 |
|---|---|---|---|---|---|
| Oleyl Erucate (Cetiol^{®} J 600) | 2,0 | | | | |
| PEG-7 Glyceryl Cocoate (Cetiol^{®} HE) | | | | | 20,0 |
| Dimethicone (Dow Corning 200) | | 0,2 | | | |
| Ceteareth-12 (Eumulgin^{®} B1) | 1,0 | | | | |
| Ceteareth-20 (Eumulgin^{®} B2) | | 0,4 | | | |
| Ceteareth-30 (Eumulgin^{®} B3) | | | | | 14,0 |
| Cetoleth-20 (Eumulgin^{®} 020) | | | | 5,0 | |
| Glycerin, Glyceryl Polyacrylate (Hispagel^{®} 200) | | | 36,7 | | |
| Lauryl Glucoside (Plantacare^{®} 1200 UP) | | | | 5,0 | |
| Laureth-7 Citrate (Plantapon^{®} LC 7) | | 0,7 | 1,0 | | |
| Glycine Soja (Soybean) Sterols (Generol^{®} 122 N) | 0,5 | | | | |
| Hydrogenated Dimer Dilinoleyl / Dimethylcarbonate Copolymer (Cosmedia^{®} DC) | 1,0 | | | | |
| Glycerin | 3,0 | | | | |
| Cocamide MEA (Comperlan® 100) | | | | 2,5 | |
| Cetrimonium Chloride (Dehyquart® A) | 3,0 | 4,0 | | | |
| Hydrolyzed Keratin (Nutrilan^{®} Keratin W) | 2,0 | | | | |
| PVP/VA (Luviskol^{®} VA 64) | | | 4,5 | | |
| PEG-90M (Polyox^{®} WSR-301) | | | 0,25 | | |
| Hydroxypropyl Methylcellulose (Methocel^{®} E4M Premium EP) | | | 0,6 | | |
| Dicocoylethyl Hydroxyethylmonium Methosulfat, Propylene Glycol (Dehyquart^{®} L 80) | | | 0,6 | | |
| Triethanolamine | | | 1,0 | | |
| CaCl₂ * 2H₂O | | 0,1 | | | |
| Ethanol | | | 12,0 | | |
| Polyquarternium-37 (Ultragel^{™} 300) | 0,2 | | | | |
| Water, Perfume, Preservatives | q.s. | q.s. | q.s. | q.s. | q.s. |

**Tabelle 17: Rinse-Off Konzepte**

| **Inhaltsstoffe (INCI)** | **1** | **2** | **3** | **4** | **5** | **6** |
|---|---|---|---|---|---|---|
| Sodium Laureth Sulfate (Texapon^{®} N 70) | 12,9 | | 12,3 | 14,3 | 14,3 | |
| Cocamidopropyl Betaine (Dehyton^{®} PK 45) | 7,7 | | 5,4 | 5,4 | 5,4 | |
| Laureth-7 Citrate (Plantapon^{®} LC 7) | 10,0 | 2,5 | | | | 10,0 |
| Guar Hydroxypropyltrimonium Chloride (Cosmedia^{®} Guar C 261 N) | | | 0,25 | 0,2 | | |
| Polyquaternium-7 | | | | 2,5 | | |
| Polyquaternium-10 | | | | | 0,15 | |
| Polyquaternium-44 | | | 1,5 | | 1,5 | |
| Glycol Distearate, Laureth-4, Cocamidopropyl Betaine (Euperlan^{®} PK 4000) | | | 10,0 | 2,0 | 2,0 | |
| PEG-40 Hydrogenated Castor Oil (Eumulgin^{®} HRE 40) | 7,5 | 7,5 | | | | |
| Mineral Oil | | | | | | 55,0 |
| (Propylheptyl Caprylate) Cetiol^{®} Sensoft | | | | | | 29,0 |
| **Propylheptyl-methylether oder Di(2-Propylheptyl)-ether** | 1,0 | 2,0 | 1,0 | 0,5 | 0,5 | 5,0 |
| Lauryl Alcohol | | | 0,5 | 0,5 | 0,5 | |
| Sodium Chloride | | | adjust viscosity | | | |
| Ethanol | | 25,0 | | | | |
| Water, Perfume, Preservatives | q.s. | q.s. | q.s. | q.s. | q.s. | q.s. |
| pH (adjusted with NaOH or citric acid) | 5,5 | 6,0 | 5,5 | 5,7 | 5,4 | 5,5 |

**Tabelle 18: Rinse-Off Konzepte**

| **Inhaltsstoffe (INCI)** | **7** | **8** | **9** | **10** | **11** | **12** |
|---|---|---|---|---|---|---|
| MIPA-Laureth Sulfate, Laureth-4, Propylene Glycol (Texapon^{®} W 90) | 40,7 | 28,3 | 28,3 | 28,3 | 28,3 | |
| Sodium Laureth Sulfate (Texapon^{®} N 70) | | | | | | 10,9 |
| Coco-Glucoside (Plantacare^{®} 818 UP) | | | | | | 6,9 |
| Laureth-7 Citrate (Plantapon^{®} LC 7) | 5,0 | 28,3 | 28,3 | 28,3 | 28,3 | |
| Laureth-2 (Mergital^{®} LM2 DEO) | 10,0 | | | | | |
| PEG-7 Glyceryl Cocoate (Cetiol^{®} HE) | 1,1 | | | | | |
| Soja Oil | | | 20,7 | | | |
| Almond Oil | | | | | | 0,5 |
| Paraffinum Liquidum | | | | | 7,0 | 23,0 |
| Cyclomethicone ((Dow Corning^{®} 245) | | | | | | |
| Dimethicone Copolyol (Dow Corning^{®} 193) | | | | 1,0 | | |
| Olus (Cegesoff^{®} PS6) | 22,0 | | | | | 10,0 |
| Propylheptyl-methylether oder Di(2-Propylheptyl)-ether | 20,0 | 41,4 | 20,7 | 40,4 | 34,4 | 15,0 |
| Acrylates Copolymer (Carbopol^{®} Aqua) | | | | | | 4,0 |
| Acrylates/C10-30 Alkyl Acrylate Crosspolymer (Pemulen^{®}TR-1) | | | | | | 0,5 |
| AMP^{®} 95 | 1,2 | | | | | |
| Poloxamer^{®} 101 | | 2,0 | 2,0 | 2,0 | 2,0 | |
| Wasser | q.s. | | | | | |

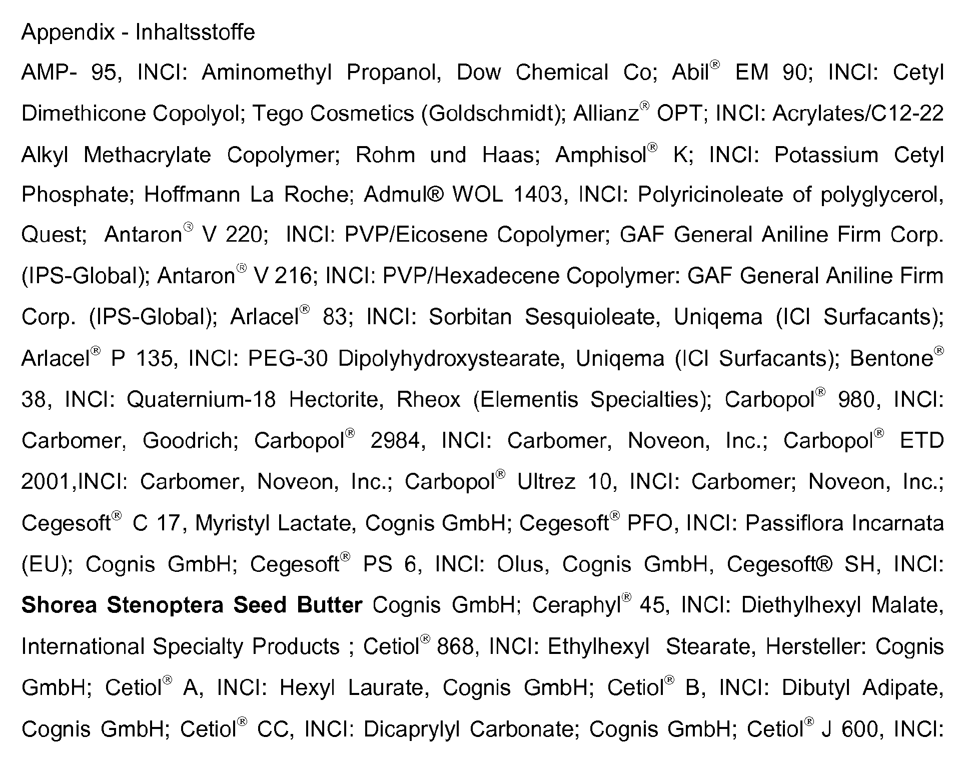

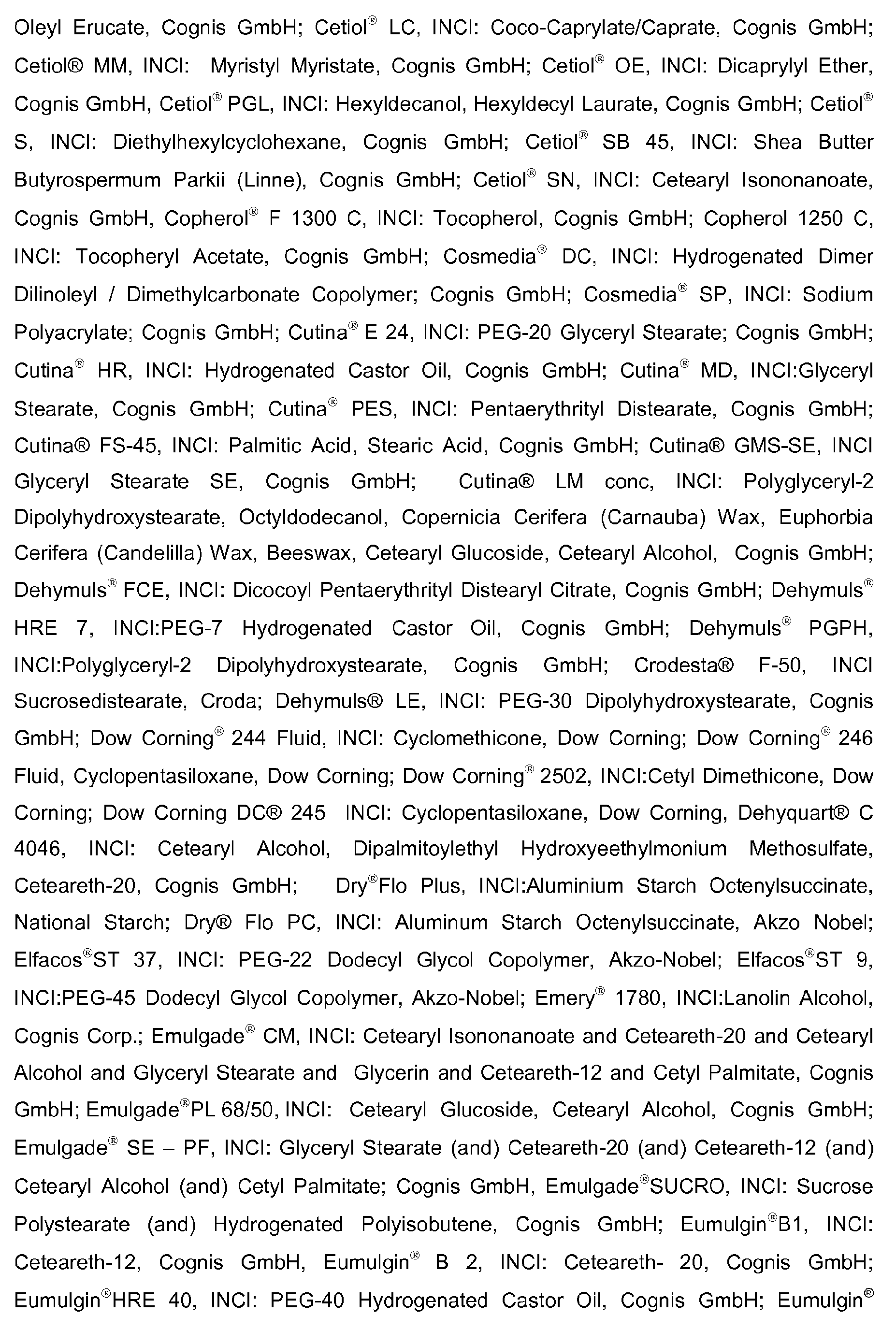

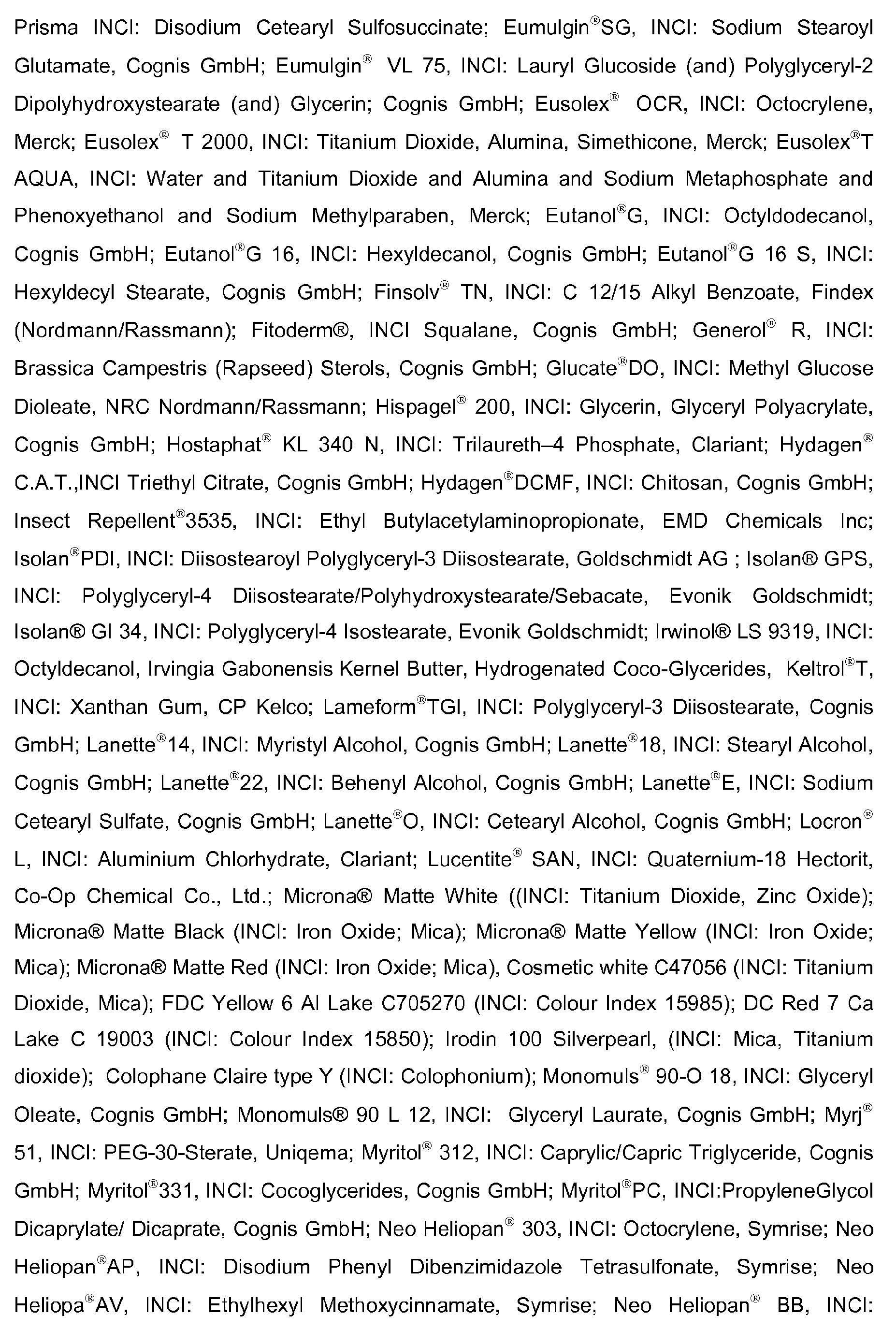

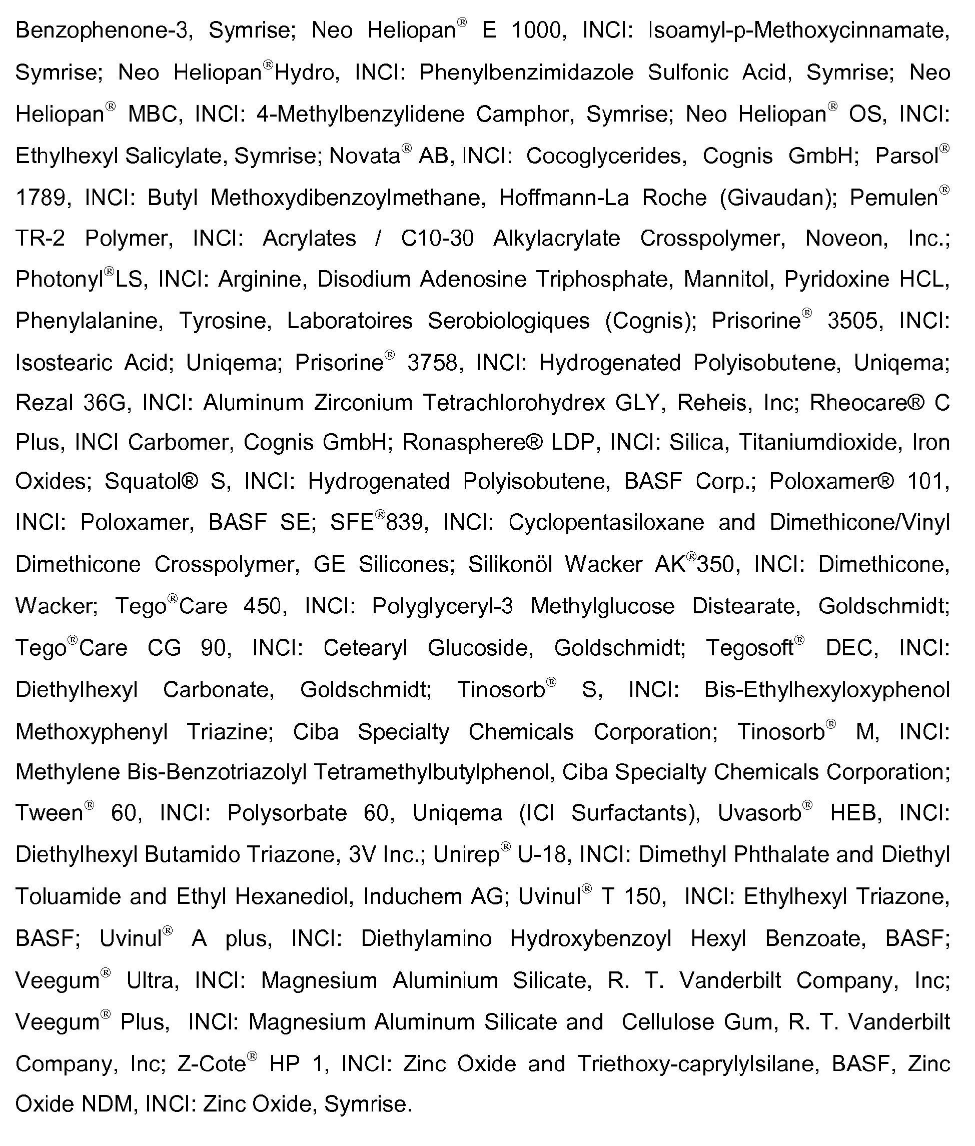

## Patentansprüche

1. Verbindungen der allgemeinen Formel (I)
R₁-O-R₂,
wobei
- R₁ einen verzweigten Alkyl(en)rest mit 10 bis 22 C-Atomen darstellt, wobei mindestens eine Verzweigung sich in Position 1, 2, 3 oder 4 zum Sauerstoffatom befindet
- R₂ einen linearen oder verzweigten Alkyl(en)rest mit 1 bis 13 C Atomen darstellt
- und R₁ und R₂ so gewählt werden, dass die Gesamtzahl der C-Atome in Formel (I) 11 bis 23 beträgt.

2. Verbindungen nach Anspruch 1, **dadurch gekennzeichnet, dass** R₁ einen verzweigten Alkyl(en)rest darstellt, wobei sich mindestens eine Verzweigung in Position 2 zur Sauerstoffgruppe befindet.

3. Verbindungen nach Anspruch 1 und/oder 2, **dadurch gekennzeichnet, dass** R₁ ausgewählt ist aus der Gruppe bestehend aus 2-Propylheptyl-, 3-Methyl-2-propyl-hexyl, 4-Methyl-2-propyl-hexyl und 5-Methyl-2-propyl-hexyl-, Trimethylheptyl-, 3,5-Dimethyloctyl-, 2,2-Dimethyloctyl-, 1-Ethyl-octyl-, 2-Ethyl-octyl, 3-Ethyl-octyl-, 4-Ethyl-octyl-, 1-Propyl-heptyl-, 3-Propyl-heptyl- und 4-Propyl-heptyl-.

4. Verbindungen nach mindestens einem der Ansprüche 1 bis 3, **dadurch gekennzeichnet, dass** R₁ und R₂ identisch sind.

5. Verbindungen nach mindestens einem der Ansprüche 1 bis 4, **dadurch gekennzeichnet, dass** R₁ und R₂ einen 2-Propylheptylrest darstellen.

6. Verwendung einer oder mehrerer Verbindungen nach einem der Ansprüche 1 bis 5 zur Herstellung von oder in kosmetischen und/oder pharmazeutischen Zubereitungen.

7. Verwendung nach Anspruch 6 als Ölkörper.

8. Kosmetische und/oder pharmazeutische Zubereitungen enthaltend in einem kosmetisch und/oder pharmazeutisch geeigneten Träger 0,1 bis 95 Gew.-% einer oder mehrerer Verbindungen nach einem der Ansprüche 1 bis 5.
